(19) **Europäisches Patentamt / European Patent Office / Office européen des brevets**

(11) **EP 3 548 095 B1**

(12) **EUROPEAN PATENT SPECIFICATION**

(45) Date of publication and mention of the grant of the patent:
**03.01.2024  Bulletin 2024/01**

(21) Application number: **16922771.7**

(22) Date of filing: **29.11.2016**

(51) International Patent Classification (IPC):
*A61K 49/00* (2006.01)   *C07K 16/28* (2006.01)
*A01K 67/027* (2006.01)   *A61K 33/24* (2019.01)
*A61P 35/00* (2006.01)   *A61K 33/243* (2019.01)

(52) Cooperative Patent Classification (CPC):
**A61K 49/0008; A01K 67/027; A61K 33/243; A61P 35/00; C07K 16/2863;** A01K 2207/12; A01K 2227/105; A01K 2267/0331; A61K 2039/505; C07K 2317/24

(86) International application number:
**PCT/CN2016/107695**

(87) International publication number:
**WO 2018/098627 (07.06.2018 Gazette 2018/23)**

(54) **METHODS OF MOUSE CLINICAL TRIAL**

VERFAHREN FÜR KLINISCHE MAUSVERSUCHE

PROCÉDÉS D'ESSAI CLINIQUE DE SOURIS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.10.2019  Bulletin 2019/41**

(73) Proprietor: **Crown Bioscience, Inc. (Taicang)**
**Jiangsu 215400 (CN)**

(72) Inventors:
• **GUO, Sheng**
 **Suzhou Industrial Park**
 **Jiangsu 215028 (CN)**
• **LI, Henry Qixiang**
 **Oceanside, California 92054 (US)**

(74) Representative: **Straus, Alexander et al**
**2K Patent- und Rechtsanwälte - München**
**Keltenring 9**
**82041 Oberhaching (DE)**

(56) References cited:
WO-A2-2012/068412    CN-A- 101 015 700
CN-A- 101 103 973    CN-A- 101 112 330
CN-A- 104 582 477    CN-A- 105 999 294

• **LIANHAI ZHANG ET AL: "A subset of gastric cancers with EGFR amplification and overexpression respond to cetuximab therapy", SCIENTIFIC REPORTS, vol. 3, no. 1, 21 October 2013 (2013-10-21), XP55705499, DOI: 10.1038/srep02992**
• **FEI DUAN ET AL: "Area under the curve as a tool to measure kinetics of tumor growth in experimental animals", JOURNAL OF IMMUNOLOGICAL METHODS, ELSEVIER SCIENCE PUBLISHERS B.V.,AMSTERDAM, NL, vol. 382, no. 1, 5 June 2012 (2012-06-05), pages 224-228, XP028399743, ISSN: 0022-1759, DOI: 10.1016/J.JIM.2012.06.005 [retrieved on 2012-06-12]**
• **TALKINGTON ANNE ET AL: "Estimating Tumor Growth Rates In Vivo", BULLETIN OF MATHEMATICAL BIOLOGY, PERGAMON PRESS, GB, vol. 77, no. 10, 19 October 2015 (2015-10-19), pages 1934-1954, XP035941828, ISSN: 0092-8240, DOI: 10.1007/S11538-015-0110-8 [retrieved on 2015-10-19]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **GIOVANI L. SILVA ET AL: "Bayesian Analysis of an Additive Survival Model with Frailty", [COMMUNICATIONS IN STATISTICS / THEORY AND METHODS] COMMUNICATIONS IN STATISTICS, vol. 33, no. 10, 2 January 2005 (2005-01-02), pages 2517-2533, XP55705330, London, GB ISSN: 0361-0926, DOI: 10.1081/STA-200031490**
- **Sun Kyung Baek ET AL: "Increased ERC C Expression C orrelates w ith Im proved O utcom e of Patients T reated w ith C isplatin as an A djuvant T herapy for C uratively Resected G astric C ancer INTRODUCTION", Cancer Res Treat, 1 January 2006 (2006-01-01), XP55705340, Retrieved from the Internet: URL:https://www.ncbi.nlm.nih.gov/pmc/artic les/PMC2741659/pdf/crt-38-19.pdf**

**Description**

FIELD OF THE INVENTION

The present invention generally relates to conducting mouse clinical trials.

BACKGROUND OF THE INVENTION

Xenograft tumors, including patient-derived xenografts (PDXs), have been used as preclinical models for efficacy evaluation of cancer drugs (Povlsen CO and Rygaard J, Pathology (1971) 79(2):159-69; Castro JE, Nature: New biology (1972) 239(90):83-4; Cobb LM, British journal of cancer (1973) 28(5):400-11). With resemblance to patient tumors in histo-/molecular pathology, PDXs have faithful pharmacological response to treatment as seen in patients (Rosfjord E et al., Biochemical pharmacology (2014) 91(2):135-43; Hidalgo M et al., Cancer discovery (2014) 4(9):998-1013; Gao H et al., Nat Med (2015) 21(11):1318-25; Owonikoko TK et al., J Transl Med (2016) 14(1):111), more so than cell line-derived xenografts (Rosfjord E et al., Biochemical pharmacology (2014) 91(2):135-43; Guo S et al., Cancer Res (2016); Tentler JJ et al., Nat Rev Clin Oncol (2012) 9(6):338-50; DeRose YS et al., Nat Med (2011) 17(11):1514-20; Zhang X et al., Cancer Res (2013) 73(15):4885-97; Zhao X et al., Neuro-oncology (2012) 14(5):574-83; Kabos P et al., Breast Cancer Res and Treatment (2012) 135(2):415-32). Availability of large collections of PDXs, representing diverse diseases (Julien S et al., Clinical Cancer Res (2012) 18(19):5314-28; Malaney P et al., Cancer Letters (2014) 344(1):1-12; Smith PG et al., Cancer Res (2014) 74(19 Supplement): 1191-91; Budinská E and Popovici V, Mol Cancer Therapeutics (2014) 12(11_Supplement):A8-A8), supports population-based preclinical mouse clinical trials (MCTs) (Gao H et al., Nat Med (2015) 21(11):1318-25; Chen D et al., Oncotarget (2015) 6(38):40815-21; Guo S et al., Oncotarget (2016); Guo S et al., Cancer Res (2016); Zhang L et al., Sci Rep (2013) 3:2992), mimicking human trials. Augmented with genomic annotations, such trial can be scientifically revealing. It would be all important and productive to have a comprehensive and scientific sound theory and methodology to guide and analyze these type of preclinical cancer pharmacology studies.

Tumor growth inhibition (TGI), as a primary tumor response endpoint in preclinical cancer pharmacology studies, may be inadequate due to its disparity with time and tumor growth rate, etc. Although other surrogate endpoints have been examined (Gao H et al., Nat Med (2015) 21(11):1318-25; Teicher BA, Progress in Drug Res (2005) 63:43-66), there is still a need for identifying ones more objectively reflective of the true drug effect. In addition, the population-based MCT study faces additional issues in its design and data analysis, where inadequate scientific theory and practice may become obstacle to hinder its broadly application. Frequently encountered questions yet to be addressed include: what scientific questions can be asked in a given MCT, number of PDXs and mice in each arm are required to answer those questions, the need of control arm, optimal readouts for assessing test subjects' drug response and survival, the impact of drug potency on study design, and method to discover predictive biomarkers, etc. (Heitjan DF et al., Cancer Res (1993) 53(24):6042-50; Wu J et al., J Biopharmaceutical Statistics (2010) 20(5):954-64; Laajala TD et al., Clinical Cancer Res (2012) 18(16):4385-96; Zhao L et al., Clinical Cancer Res (2011) 17(5):1057-64; Xia C et al., J Biometrics & Biostatistics (2013); Pierrillas PB et al., The AAPS J (2016); Choudhury KR et al., Statistics in Med (2010) 29(23):2399-409). Therefore, there is continuing needs develop new methodology for conducting mouse clinical trial.

BRIEF SUMMARY OF THE INVENTION

The invention is defined by the appended claims. Embodiments which do not fall under scope of the claims do not form part of the invention.

In one aspect, the present disclosure provides a method of conducting mouse clinical trial comprising the steps of:

> receiving a dataset of tumor volumes measured in a mouse clinical trial, wherein the mouse clinical trial comprises the steps of:

>> obtaining a tumor sample derived from a patient;
>> grafting the tumor sample to a treatment group comprising m mice and a control group comprising n mice, wherein m and n are integers;
>> administering a drug to the treatment group;
>> administering a vehicle to the control group; and
>> measuring tumor volume of the treatment group and tumor volume of the control group at a plurality of days;

> determining tumor growth curve of the treatment group and tumor growth curve of the control group;
> determining area under curve (AUC) of the treatment group ($AUC_T$) and AUC of the control group ($AUC_C$) ; and

evaluating efficacy of the drug based on an AUC ratio between the $AUC_T$ and the $AUC_C$.

$$AUC_T = \int_0^{d(T)} \ln V_x^{(T)}\, dx - d(T) \times \ln V_0^{(T)}$$ , wherein $V_0^{(T)}$ is the tumor volume of the treatment group at day 0, $V_x^{(T)}$ is the tumor volume of the treatment group at day x, d(T) is the number of days the measuring step for the treatment group lasts; and $$AUC_C = \int_0^d \ln V_x^{(C)}\, dx - d \times \ln V_0^{(C)}$$ , wherein $V_0^{(C)}$ is the tumor volume of the control group at day 0, $V_x^{(C)}$ is the tumor volume of the control group at day x, d(C) is the number of days the measuring step for the treatment group lasts.

In certain embodiments, d(T) = d(C), wherein the AUC $$\text{ratio} = \frac{AUC_T}{AUC_C}$$ .

In certain embodiments, d(T) = d(C), wherein the AUC $$\text{ratio} = \frac{AUC_T/d(T)^2}{AUC_C/d(C)^2}$$ .

In certain embodiments, $m \geq 3$ and $n \geq 3$. In certain embodiments, $m = n$.

In certain embodiments, the evaluating step comprises determining that the drug is effective when the AUC ratio is less than 1.

In certain embodiments, the method disclosed herein further comprises the step of determining correlation of a factor to the efficacy of the drug.

In certain embodiments, the factor is expression level of a gene. In one embodiment, the gene is EGFR and the drug is cetuximab.

In certain embodiments, the tumor volumes are between 100-300 $mm^3$.

In another aspect, the present disclosure provides a method of conducting mouse clinical trial comprising the steps of:

receiving a dataset of tumor volumes measured in a mouse clinical trial, wherein the mouse clinical trial comprises the steps of:

obtaining a tumor sample derived from a patient;
grafting the tumor sample to a treatment group comprising m mice and a control group comprising n mice, wherein m and n are integers;
administering a drug to the treatment group;
administering a vehicle to the control group; and
measuring tumor volume of the treatment group and tumor volume of the control group at a plurality of days;

evaluating impact of a factor on the tumor volume of the treatment group, wherein the tumor volume of the treatment group after logarithmic transformation has a linear relationship with the factor.

In certain embodiments, the factor is selected from the group consisting of: efficacy of the drug, cancer type of the patient, expression level of a gene, and existence of a mutation.

In one embodiment, the drug is cisplatin.

In certain embodiments, the cancer type is esophageal cancer, gastric cancer or lung cancer.

In once embodiments, the gene is ERCC.

In some embodiments, $m \geq 3$ and $n \geq 3$. In some embodiment, $m = n$.

In certain embodiments, the method disclosed herein further comprises conducting a second mouse clinical trial based on the impact of the factor determined in the evaluation step.

In yet another aspect, the present disclosure provides a method of conducting a mouse clinical trial, comprising

receiving a dataset of tumor volumes in a mouse clinical trial, wherein the mouse clinical trial comprises the steps of:

obtaining a plurality of tumor samples derived from a plurality of patients;
grafting the plurality of tumor samples to a treatment group of mice and a control group of mice;
administering a drug to the treatment group;
administering a vehicle to the control group; and

measuring tumor volume of the treatment group and tumor volume of the control group at a plurality of days;

determining progress-free survival (PFS) or overall survival (OS) of the treatment group and the control group;
evaluating efficacy of the drug based on the PFS or OS using an additive frailty model.

In certain embodiments, the additive frailty model includes a hazard function, wherein the hazard function for the $j$-th mouse of the i-th PDX is

$$h_{ij}(t) = h_0(t)\exp(u_i + (w + v_i)T_{ij} + \beta^{\mathrm{T}}X_i)$$

wherein $h_0(t)$ is a baseline hazard function, $u_i$ is a random effect associated with the i-th patient without drug treatment, w is mean drug effect, $v_i$ is a random effect associated the i-th patient that depicts drug response deviating from $w$, $T_{ij}$ is 0 for the control group and 1 for the treatment group, $X_i$ is a vector for a covariate of the plurality of patients, $\beta^{\mathrm{T}}$ is a vector quantifying fixed effects of the covariate.

In certain embodiments, the PFS is the time until the tumor volume doubles.

In certain embodiments, the covariate is cancer type, expression of a gene or a gene mutation.

In certain embodiments, the treatment group consists of n mice for each patient and the control group consists of n mice for each patient, wherein n $\geq$ 3.

In certain embodiments, the method disclosed herein further comprises conducting a second mouse clinical trial based on the hazard function.

BRIEF DESCRIPTION OF THE FIGURES

Figure 1 illustrates tumor volume doubling time (DT) in PDXs for 10 cancers ranked by increasing median DT. LI: liver, BR: breast, PA: pancreatic, OV: ovarian, CR: colorectal, LU: lung, ES: esophageal, GL: gallbladder, GA: gastric, HN: head and neck.

Figure 2 illustrates that in drug groups, the coefficient of determination ($R^2$) by Equation 2 is smallest when the TV ratio between end and start days is around 1. When the TV ratio is larger than 2 and 3, there are 91.8% and 95.1% growth curves with $R^2 >0.70$.

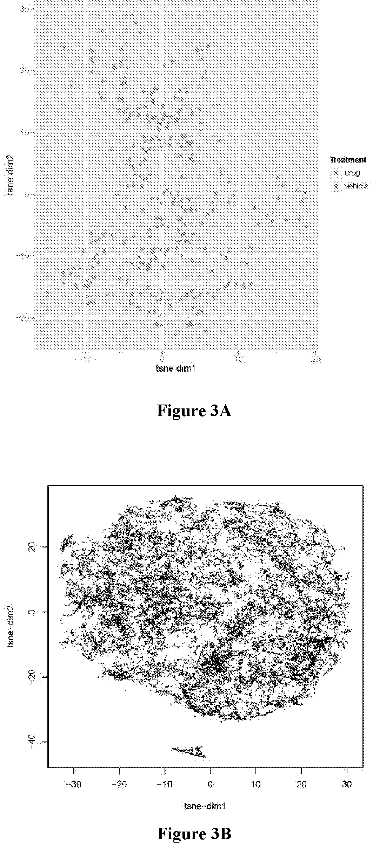

Figure 3A illustrates the relative distance of growth curves under drug treatment and vehicle treatment, visualized by the first two t-SNE dimensions (van der Maatern & Hintton, J machine Learning Res (2008) 2579-2605). A total of 300 randomly selected growth curves were used including 250 under drug treatment and 50 under vehicle treatment, close to the ratio in the total set of 34081 growth curves. For each growth curve, its 480x480 jpeg file was converted into a numeric matrix of the same dimension and used as input to the R Rtsne package (Krijthe J., CRAN (2015)). About 20% of growth curves under drug treatment form a separated cluster in the upper part of the graph.

Figure 3B illustrates the visualization of all 34081 growth curves. The isolated cluster includes irregular growth curves that are not linear, or have V-like or Λ-like shapes.

Figure 4A illustrates the variation of TGI by day and by drug efficacy in PDXs with fastest growth rate, i.e. $k_C$, based on Equation 4 with $k_T \leq k_C$. The red lines are TGIs for $k_T/k_C$ ranging from 0 to 1 with 0.1 stepwise increase from bottom to top; the blue lines are TGIs for $k_T/k_C$ ranging from 2 to 1 with 0.1 stepwise decrease from top to bottom. Both red and blue lines are drawn with slight separation for TGI=1. Gray portion of the lines means that the tumor volumes are larger than 3000mm$^3$, at which mice are sacrificed and TGIs no longer exist.

Figure 4B illustrates the variation of TGI by day and by drug efficacy in PDXs with median growth rate, i.e. $k_C$, based on Equation 4 with $k_T \leq k_C$. The red lines are TGIs for $k_T/k_C$ ranging from 0 to 1 with 0.1 stepwise increase from bottom to top; the blue lines are TGIs for $k_T/k_C$ ranging from 2 to 1 with 0.1 stepwise decrease from top to bottom. Both red and blue lines are drawn with slight separation for TGI=1. Gray portion of the lines means that the tumor volumes are larger than 3000mm$^3$, at which mice are sacrificed and TGIs no longer exist.

Figure 4C illustrates the variation of TGI by day and by drug efficacy in PDXs with slowest growth rate, i.e. $k_C$, based on Equation 4 with $k_T \leq k_C$. The red lines are TGIs for $k_T/k_C$ ranging from 0 to 1 with 0.1 stepwise increase from bottom to top; the blue lines are TGIs for $k_T/k_C$ ranging from 2 to 1 with 0.1 stepwise decrease from top to bottom. Both red and blue lines are drawn with slight separation for TGI=1. Gray portion of the lines means that the tumor volumes are larger than 3000mm$^3$, at which mice are sacrificed and TGIs no longer exist.

Figure 5A illustrates TGI at day 3, 7, 10, 14, 17, and 21 for 40 PDXs in a MCT. The average starting tumor volumes are between 115mm$^3$~194mm$^3$, and differ by 4.7% on average between the drug and vehicle groups for a PDX. TGI is calculated by Equation 3.

Figure 5B illustrates TGIs at 6 measurement days for the first 4 PDXs in a MCT as shown in Figure 5A. TGI increases by day when it is between 0 and 1. A strong positive correlation is observed between tumor growth rate $k_c$ and TGI at day 17 for 24 PDXs whose TGIs are between 0 and 1 for all measurement days.

Figure 5C illustrates when between 0 and 100%, TGI is positive correlated with vehicle tumor growth rate $k_c$. The graphs show a set of 24 PDXs with TGIs less than 100% at all 4 measurement days from Figure 5A.

Figure 5D illustrates TGI at day 3, 7, 10, 14, 17, and 20 for 38 PDXs in a second MCT. TGI is calculated by Equation 3.

Figure 5E illustrates TGIs at 6 measurement days for the first 4 PDXs in the second MCT as illustrated in Figure 5D. TGI decreases by day when it is greater than 1. A strong negative correlation is observed between tumor growth rate $k_c$ and TGI at day 17 for 18 PDXs whose TGIs are greater than 1 at all measurement days.

Figure 5F illustrates when greater than100%, TGI is negatively correlated with vehicle tumor growth rate $k_c$. The graphs show a set of 18 PDXs with TGI greater than 100% at all 4 measurement days from Figure 5D.

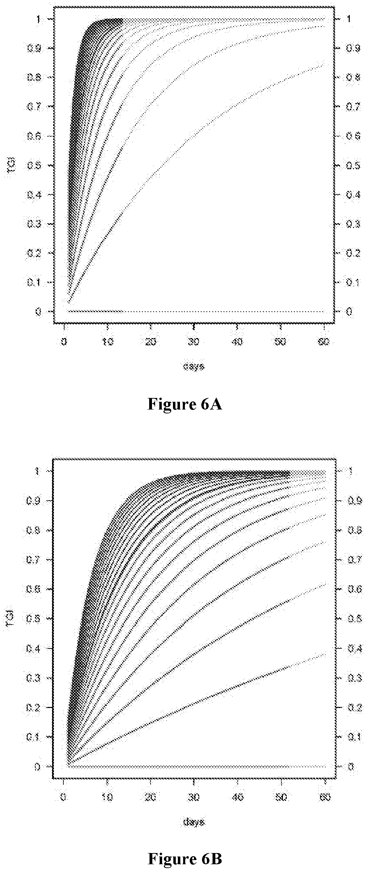

Figure 6A illustrates TGI is a growth rate biased and time-dependent efficacy metric. The variation of TGI by day and by drug efficacy in PDXs with fastest growth rate, i.e. $k_C$, based on Equation $TGI = 1 - \dfrac{e^{k_T x}}{e^{k_C x}}$ with $k_T \leq k_C$. The red lines are TGIs for $k_T/k_C$ ranging from 0 to 1 with 0.1 stepwise increase from bottom to top, the blue lines are TGIs for $k_T/k_C$ ranging from 2 to 1 with 0.1 stepwise decrease from top to bottom. Both red and blue lines are drawn with slight separation for $k_T = k_C$. Gray portion of the lines means that the tumor volumes are larger than 3000mm$^3$, at which mice are sacrificed and TGIs no longer exist.

Figure 6B illustrates TGI is a growth rate biased and time-dependent efficacy metric. The variation of TGI by day and by drug efficacy in PDXs with median growth rate, i.e. $k_C$, based on Equation $TGI = 1 - \dfrac{e^{k_T x}}{e^{k_C x}}$ with $k_T \leq k_C$. The red lines are TGIs for $k_T/k_C$ ranging from 0 to 1 with 0.1 stepwise increase from bottom to top, the blue lines are TGIs for $k_T/k_C$ ranging from 2 to 1 with 0.1 stepwise decrease from top to bottom. Both red and blue lines are drawn with slight separation for $k_T = k_C$. Gray portion of the lines means that the tumor volumes are larger than 3000mm$^3$, at which mice are sacrificed and TGIs no longer exist.

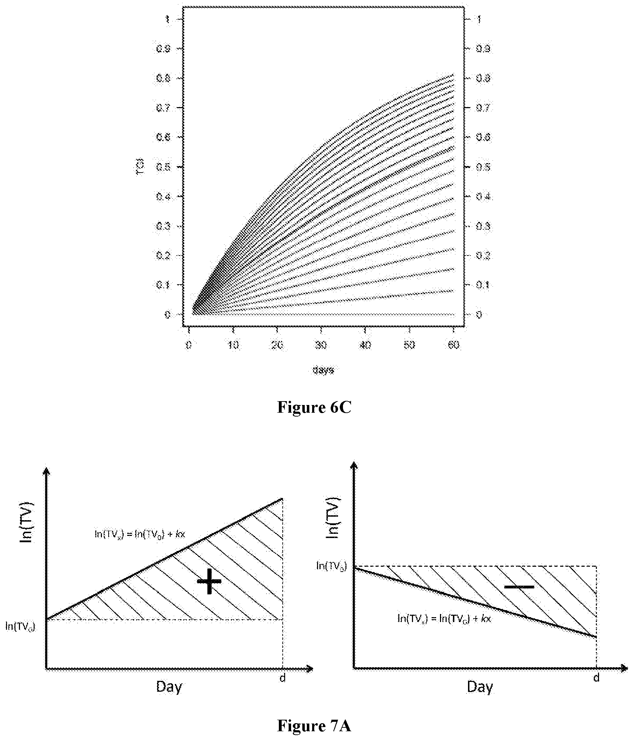

Figure 6C illustrates TGI is a growth rate biased and time-dependent efficacy metric. The variation of TGI by day and by drug efficacy in PDXs with slowest growth rate, i.e. $k_C$, based on Equation $TGI = 1 - \dfrac{e^{k_T x}}{e^{k_C x}}$ with $k_T \leq k_C$. The red lines are TGIs for $k_T/k_C$ ranging from 0 to 1 with 0.1 stepwise increase from bottom to top, the blue lines are TGIs for $k_T/k_C$ ranging from 2 to 1 with 0.1 stepwise decrease from top to bottom. Both red and blue lines are drawn with slight separation for $k_T = k_C$. Gray portion of the lines means that the tumor volumes are larger than 3000mm$^3$, at which mice are sacrificed and TGIs no longer exist.

Figure 7A is chematic illustrations of AUC. AUC is positive when tumor grows, and is negative when tumor shrinks.

Figure 7B illustrates efficacy end points of TGI for 50 PDXs in a MCT, each PDX has 10 mice each in the vehicle group and the drug group.

Figure 7C illustrates efficacy end points of AUC for 50 PDXs in the MCT as shown in Figure 7B, each PDX has 10 mice each in the vehicle group and the drug group.

Figure 7D illustrates the bootstrap 90% confidence interval sizes of AUC ratios under n:n design, based on efficacy data for 50 PDXs in the MCT as shown in Figure 7B and 7C with 10 mice each in the vehicle group and the drug group.

Figure 7E illustrates the bootstrap 90% confidence interval sizes for AUC ratios in a MCT of one embodiment. The n:n design means there are n mice in both the vehicle group and the drug group.

Figure 7F illustrates the bootstrap 90% confidence interval sizes for AUC ratios in a MCT of one embodiment. The n:n design means there are n mice in both the vehicle group and the drug group.

Figure 7G illustrates the bootstrap 90% confidence interval sizes for AUC ratios in a MCT of one embodiment. The n:n design means there are n mice in both the vehicle group and the drug group.

Figure 7H illustrates the bootstrap 90% confidence interval sizes for AUC ratios in a MCT of one embodiment. The n:n design means there are n mice in both the vehicle group and the drug group.

Figure 8A illustrates the correlation between EGFR expression and efficacy end points of TGI in a set of 27 gastric cancer PDXs treated with cetuximab.

Figure 8B illustrates the correlation between EGFR expression and efficacy end points of AUC ratio in the set of 27 gastric cancer PDXs treated with cetuximab as shown in Figure 8A.

Figure 9A illustrates the structure of the clustered longitudinal data for a PDX in a MCT. PDX level and mouse level covariates can be incorporated into the models.

Figure 9B illustrates the growth curve of 42 PDXs under vehicle treatment.

Figure 9C illustrates the growth curve of 42 PDXs under cisplatin treatment. Each mouse received cisplatin at 4mg/kg, daily for 3 weeks.

Figure 9D illustrates the fitted tumor growth curves for 3 cancers under vehicle treatment and drug treatment by Equation 7.

Figure 9E illustrates the fitted growth curves for gastric cancer PDXs with highest and lowest ERCC1 mRNA expression by Equation 8.

Figure 9F illustrates the power curves at significance level a=0.05 when treatment reduces tumor growth rate by 10% and 20%, i.e. $\beta_1/\beta_2$ = -0.1 and -0.2 in Equation 9. The 10 colored curves in each graph denote the number of mice for every PDX.

Figure 9G illustrates the power curves at significance level a=0.05 when treatment reduces tumor growth rate by 10% to 90%, i.e. $\beta_1/\beta_2$=-0.1 to -0.9 in Equation 9. The 10 colored curves in each graph denote the number of mice for every PDX.

Figure 10A illustrates that the median PFS values for PDXs under vehicle treatment and cisplatin treatment are highly correlated. The graphs use data from the cisplatin dataset as shown in Figure 9B-9C.

Figure 10B illustrates that the median OS values for PDXs under vehicle treatment and cisplatin treatment are highly correlated. The graphs use data from the cisplatin dataset as shown in Figure 9B-9C.

Figure 11A illustrates the survival curves under vehicle and drug treatments for PFS end points.

Figure 11B illustrates the survival curves under vehicle and drug treatments for OS end points.

Figure 11C illustrates the first frailty term $u_i$ in Equation 10 is positively correlated with the tumor growth rate $k_c$ in OS survival analysis.

Figure 11D illustrates the first frailty term $u_i$ in Equation 10 is positively correlated with the tumor growth rate $k_c$ in PFS survival analysis.

Figure 11E illustrates the power curves at significance level a=0.05 when the hazard ratio is 0.5 for OS survival analysis. The 10 colored curves in each graph denote the number of mice for every PDX.

Figure 11F illustrates the power curves at significance level a=0.05 when the hazard ratio is 0.4 for OS survival analysis. The 10 colored curves in each graph denote the number of mice for every PDX.

Figure 11G illustrates the power curves at significance level a=0.05 when the hazard ratio is between 0.1 and 0. 9 in PFS analysis for the cisplatin dataset. The 10 colored curves in each graph denote the number of mice for every PDX.

Figure 11H illustrates the power curves at significance level a=0.05 when the hazard ratio is between 0.1 and 0. 9 in OS analysis for the cisplatin dataset. The 10 colored curves in each graph denote the number of mice for every PDX.

## DETAILED DESCRIPTION OF THE INVENTION

In the Summary of the Invention above and in the Detailed Description of the Invention, and the claims below, and in the accompanying drawings, reference is made to particular features (including method steps) of the invention. It is to be understood that the disclosure of the invention in this specification includes all possible combinations of such particular features. For example, where a particular feature is disclosed in the context of a particular aspect or embodiment of the invention, or particular claim, that feature can also be used, to the extent possible, in combination with and/or in the context of other particular aspects and embodiments of the invention, and in the invention generally.

The term "comprises" and grammatical equivalents thereof are used herein to mean that other components, ingredients, steps, etc. are optionally present. For example, an article "comprising" (or "which comprises") components A, B, and C can consist of (i.e., contain only) components A, B, and C, or can contain not only components A, B, and C but also one or more other components.

Where reference is made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where the context excludes that possibility).

Where a range of value is provided, it is understood that each intervening value, to the tenth of the unit of the lower limit unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, the embodiments described herein can be practiced without there specific details. In other instances, methods, procedures and components have not been described in detail so as not to obscure the related relevant function being described. Also, the description is not to be considered as limiting the scope of the implementations described herein. It will be understood that descriptions and characterizations of the embodiments set forth in this disclosure are not to be considered as mutually exclusive, unless otherwise noted.

A mouse clinical trial (MCT) is a population-based efficacy trial mimicking a human trial. It can be used to understand molecular mechanisms of drugs, to identify and prioritize indications, to reposition drugs, to guide clinical trials by discovering and evaluating biomarkers or assessing combination therapies. MCTs offer several advantages over human trials: reducing/removing much variance frequently encountered in human trials (e.g. prior treatments, individual health/nutrition conditions, other medications, heterogeneity in metabolisms and toxicity); less regulatory/ethic constraint for scientific exploration; consistent drug exposure and reducing PK by using inbred mice; highly characterized PDXs with potentially historic data and high translatability; time saving and cost effective. The most distinct feature of MCTs is that PDX avatars of a single patient can join multiple treatment arms simultaneously, thus greatly enhancing the power of MCTs when properly designed and analyzed.

The present disclosure provides new methods for the design and analysis of both single-PDX studies and MCTs on a cohort of PDXs. In certain embodiments, the methods disclosed herein are based on an exponential growth model. Many mathematical models were proposed to describe tumor growth (Zhao et al., Clinical cancer research (2011) 17:1057-1064; Benzekry et al., PloS computational biology (2014) 10:e1003800), but might not be particularly advantageous in practice at the expense of requiring more parameters and data points. Furthermore, these models are largely unsuitable, or overly data-dependent, for data analysis in MCTs when tumor growth does not follow exponential kinetics, e.g. ones with V- or $\Lambda$-shapes. The major advantage of the exponential growth model lies in its simplicity, interpretability, and linearity after a logarithmic transformation, which is supported by our large collection of PDX trial datasets. In certain embodiment, the tumor growth rate $k$, and use $k_T/k_C$ are calculated as an objective measure of tumor response to treatment. In certain embodiments, AUC ratio is used as a new tumor response endpoint superior to the conventionally used TGI. The AUC ratio is also suitable for immune-oncology pharmacology studies, in which large efficacy variations are observed. Defined as the median of ratios, this metric is in general not sensitive to extreme values and more robust than TGI.

In one aspect, the present disclosure provides a method for analyzing MCT data using AUC ratio, which is a measure of average growth rate ratio, and can be directly utilized to assess objective response rate (ORR). AUC ratio also measures accumulative tumor response even if a growth curve is not exponential.

In another aspect, the present disclosure provides a method for analyzing MCT data using linear mixed model (LMM). The LMM-based method can process nearly all MCT data, using quadratic terms of time if necessary. It can distinguish prognostic and predictive biomarkers, quantify different drug response of multiple cancers, and model heterogeneity of PDXs and mice.

In another aspect, the present disclosure provides a method for analyzing MCT data using additive frailty model. Like LMM, the additive frailty model takes into account of the frailty or heterogeneity of PDX's growth and drug response, as well as the clustering structure of PDXs and mice. Therefore, it gives more accurate estimate on hazard ratios and p-values than the proportional hazard Cox model, or the Kaplan-Meier method and the ordinary log-rank test.

The methods disclosed herein are not exclusive of each other, nor are they the only forms of corresponding analysis. In certain embodiments, the method uses a 2-level LMM by treating PDXs as the level-2 clusters and mice within PDX as the level-1 unit of analysis, where the dependent variable is the normalized AUC value. This is particularly suitable for irregular growth curves that cannot be satisfactorily modelled by the 3-level LMMs, without resorting to more complex methods like the piecewise modeling approaches. It is also possible to jointly model the longitudinal and survival data. If there is no significant difference between PDX in drug response, the additive frailty model can be reduced to the shared frailty model. If a MCT is conducted at multiple sites, the method can use a 4-level LMM to capture the site variance should it not be negligible. Also, for the survival analysis, the definition of PFS and OS can vary by MCTs. For example, in leukemia PDXs, the end points can be defined as in human trials. Naturally, these methods can be used for single-PDX studies if the number of mice in each treatment group is sufficiently large.

In one embodiment, the method provides statistical power simulation using LMM that gives concrete recommendations on trial design. In particular, it answers the frequently asked question on how many PDXs and how many mice per PDX to use, and gives flexibility on the PDX and mouse number combinations. The power analysis results on both LMM and survival analysis advise cautions for using the "one mouse per PDX per treatment" paradigm, particularly in small scale MCTs. With proper adaption and extension, methods disclosed herein can make the design and analysis of MCTs more rational and more powerful.

In one aspect, the present disclosure provides a method of conducting mouse clinical trial comprising the steps of:

receiving a dataset of tumor volumes measured in a mouse clinical trial, wherein the mouse clinical trial comprises the steps of:

obtaining a tumor sample derived from a patient;

grafting the tumor sample to a treatment group comprising m mice and a control group comprising n mice, wherein m and n are integers;
administering a drug to the treatment group;
administering a vehicle to the control group; and
measuring tumor volume of the treatment group and tumor volume of the control group at a plurality of days;

determining tumor growth curve of the treatment group and tumor growth curve of the control group;
determining area under curve (AUC) of the treatment group ($AUC_T$) and AUC of the control group ($AUC_C$) ; and
evaluating efficacy of the drug based on an AUC ratio between the $AUC_T$ and the $AUC_C$.

As used herein, "mouse clinical trial" refers to research studies that test how well new medical approaches work by using mice to mimic human diseases. In certain embodiments, the mice used mimic tumor. In certain embodiments, the mice used have patient derived xenograft (PDX). "Patient derived xenograft," as used herein, refers to a graft of tissue or cells taken from a human patient donor, and grafted into an animal model (e.g., mouse, rat, rabbit, etc.). In some embodiments, the xenograft tissue or cells are tumor tissue or cells, or cancerous tissue or cells. In some embodiments, the xenograft is pre-treated before grafting into the animal model. The term "pre-treated" when refers to tissue, generally relates to any processing methods known in the art to treat a tissue before its engraftment, such as washing, homogenization, re-suspension and mixing with a solution (e.g., saline, PBS etc.) or a matrix (e.g., collagen). The term "pre-treated" when refers to cells, includes any processing methods known in the art to treat cells before its engraftment, such as culture, subculture, activating, treatment with an agent, centrifugation, re-suspension, filtration, and mixing with a solution (e.g., saline, PBS etc.) or a matrix (e.g., collagen). After grafted with xenograft, the animal model is allowed sufficient time to develop a lesion of the human disease for further use. The xenograft can be grafted to the animal model using any suitable methods known in the art, for example, by grafting cells subcutaneously, intraperitoneally, or intravenously through injection; or alternatively, by implanting a fraction of tissue through surgery. In some embodiments, the xenografts are tumor cells or cancerous cells, and are grafted to the animal model through subcutaneously injection.

As used herein, the term "tumor" or "cancer" refers to a group of diseases involving abnormal cell growth and division. In general, cancers can be categorized according to the tissue or organ from which the cancer is located or originated and morphology of cancerous tissues and cells. As used herein, cancer types include, without limitation, acute lymphoblastic leukemia (ALL), acute myeloid leukemia, adrenocortical carcinoma, anal cancer, astrocytoma, childhood cerebellar or cerebral, basal-cell carcinoma, bile duct cancer, bladder cancer, bone tumor, brain cancer, cerebellar astrocytoma, cerebral astrocytoma/malignant glioma, ependymoma, medulloblastoma, supratentorial primitive neuroectodermal tumors, visual pathway and hypothalamic glioma, breast cancer, Burkitt's lymphoma, ervical cancer, chronic lymphocytic leukemia, chronic myelogenous leukemia, colon cancer, emphysema, endometrial cancer, ependymoma, esophageal cancer, Ewing's sarcoma, retinoblastoma, gastric (stomach) cancer, glioma, head and neck cancer, heart cancer, Hodgkin lymphoma, islet cell carcinoma (endocrine pancreas), Kaposi sarcoma, kidney cancer (renal cell cancer), laryngeal cancer, leukaemia, liver cancer, lung cancer, neuroblastoma, non-Hodgkin lymphoma, ovarian cancer, pancreatic cancer, pharyngeal cancer, prostate cancer, rectal cancer, renal cell carcinoma (kidney cancer), retinoblastoma, Ewing family of tumors, skin cancer, stomach cancer, testicular cancer, throat cancer, thyroid cancer, vaginal cancer.

The term "tumor sample" or "cancer sample" used herein encompasses any sample obtained, directly or indirectly, from a tumor or cancer patient. A sample can, by way of non-limiting example, include cerebrospinal fluid (CSF), blood, amniotic fluid, sera, urine, feces, epidermal sample, skin sample, cheek swab, sperm, amniotic fluid, cultured cells, bone marrow sample and/or chorionic villi. Cancer cell cultures can also be used as samples. A cancer sample can also be, e.g., a sample obtained from any organ or tissue (including a surgical removal, biopsy or autopsy specimen), can comprise cells (whether primary cells or cultured cells), medium conditioned by any cell, tissue or organ, tissue culture. In some embodiments, biological samples suitable for the invention are samples which have been processed to release or otherwise make available a nucleic acid for detection as described herein. Suitable biological samples may be obtained from a stage of life such as a fetus, young adult, adult (e.g., pregnant women), and the like. Fixed or frozen tissues also may be used.

The "drug" used herein can be any chemical or medical approach that is tested in the mouse clinical trial. In certain embodiments, the drug used in the method is a potential drug for treating tumor or cancer. Examples of tumor/cancer drugs include, without limitation, Abritrexate, Afatinib dimaleate, Afinitor, Alecensa, Alectinib, Alimta, Araxane, Avastin, Bevacizumab, Blenoxane, Bleomycin, Camptosar, Capecitabine, Carboplatin, Ceritinib, Cetuximab, Cyramza, Docetaxel, Eloxatin, Erbitux, Erlotinib, Everolimus, 5-FU, Filotrif, Folex, Folex PFS, Fluorouracil Injection, Gefitinib, Gemcitabine Hydrochloride, Gemzar, Hydrea, Hydroxyurea, Iressa, Irinotecan hybdrochloride, Keytruda, Leucovorin Calcium, Lonsurf, Methotrxate, Methotrexate LPF, Mexate, Mexate-AQ, Mustargen, Navelbine, Necitumumab, Nivolumab, Opdivo, Osimertinib, Oxaliplatin, Paclitaxel, Panitumumab, Paraplat, Paraplatin, Pembrolizumab, Pemetrexed disoldium, Protrazza, Ramucirumab, Regorafenib, Stivarga, Tagrisso, Tarceva, Taxol, Taxotere, Trifluridine and Tipiracil ydrochloride, Vectibix, Wellcovorin, Xalkori, Xeloda, Zatrap, Ziv-aflibercept, Zykadia.

As used herein, a "vehicle" refers to a preparation that has no effect to treat the disease. Typically, the preparation does not include the drug being tested. In certain embodiments, the vehicle is a sham preparation (e.g., a placebo), either an excipient-only preparation or a sugar pill preparation.

As used herein, "administering" or "administration of" a drug or therapeutic to a subject (and grammatical equivalents of this phrase) includes both direct administration, including self-administration, directly into or onto a target tissue or to administer a therapeutic to a subject whereby the therapeutic positively impacts the tissue to which it is targeted, and indirect administration, including the act of prescribing a drug. The administration can be subcutaneous, intravenous, intramuscular, intra-articular, intra-synovial, intrasternal, intrathecal, intrahepatic, intraventricular, intragastrical, intralesional and intracranial injection or infusion techniques. In some embodiments, the drugs are administered intravenously, subcutaneously, orally, intramuscularly, intraventricularly, intragastrically, or by inhalation.

The term "tumor growth curve" used herein refers to an empirical model of tumor volume change over time. In certain embodiment, the tumor volume is measured every one, two, three or four days.

In certain embodiments, $\mathrm{AUC_T} = \int_0^{d(T)} \ln V_x^{(T)}\, dx - \mathrm{d(T)} \times \ln V_0^{(T)}$, wherein $V_0^{(T)}$ is the tumor volume of the treatment group at day 0, $V_x^{(T)}$ is the tumor volume of the treatment group at day x, d(T) is the number of days the measuring step for the treatment group lasts.

In certain embodiments, $\mathrm{AUC_C} = \int_0^{d} \ln V_x^{(C)}\, dx - \mathrm{d} \times \ln V_0^{(C)}$, wherein $V_0^{(C)}$ is the tumor volume of the control group at day 0, $V_x^{(C)}$ is the tumor volume of the control group at day x, d(C) is the number of days the measuring step for the treatment group lasts.

In certain embodiments, d(T) = d(C), wherein the AUC $\mathrm{ratio} = \frac{AUC_T}{AUC_C}$.

In certain embodiments, d(T) = d(C), wherein the AUC $\mathrm{ratio} = \frac{AUC_T/d(T)^2}{AUC_C/d(C)^2}$.

In certain embodiments, $m \geq 3$ and $n \geq 3$. In certain embodiments, m = n.

In certain embodiments, the evaluating step comprises determining that the drug is effective when the AUC ratio is less than 1.

In certain embodiments, the method disclosed herein further comprises the step of determining correlation of a factor to the efficacy of the drug. In certain embodiments, the factor is selected from the group consisting of: efficacy of the drug, cancer type of the patient, expression level of a gene, and existence of a mutation.

In certain embodiments, the factor is expression level of a gene. In one embodiment, the gene is EGFR and the drug is cetuximab.

In certain embodiments, the tumor volumes are between 100-300 mm$^3$.

In another aspect, the present disclosure provides a method of conducting mouse clinical trial comprising the steps of:

receiving a dataset of tumor volumes measured in a mouse clinical trial, wherein the mouse clinical trial comprises the steps of:

obtaining a tumor sample derived from a patient;
grafting the tumor sample to a treatment group comprising m mice and a control group comprising n mice, wherein m and n are integers;
administering a drug to the treatment group;
administering a vehicle to the control group; and
measuring tumor volume of the treatment group and tumor volume of the control group at a plurality of days;

evaluating impact of a factor on the tumor volume of the treatment group, wherein the tumor volume of the treatment group after logarithmic transformation has a linear relationship with the factor.

In certain embodiments, the factor is selected from the group consisting of: efficacy of the drug, cancer type of the patient, expression level of a gene, and existence of a mutation.

In one embodiment, the drug is cisplatin.

In certain embodiments, the cancer type is esophageal cancer, gastric cancer or lung cancer.

In once embodiments, the gene is ERCC.

In some embodiments, m ≥ 3 and n ≥ 3. In some embodiment, m = n.

In certain embodiments, the method disclosed herein further comprises conducting a second mouse clinical trial based on the impact of the factor determined in the evaluation step.

In yet another aspect, the present disclosure provides a method of conducting a mouse clinical trial, comprising

receiving a dataset of tumor volumes in a mouse clinical trial, wherein the mouse clinical trial comprises the steps of:

obtaining a plurality of tumor samples derived from a plurality of patients;
grafting the plurality of tumor samples to a treatment group of mice and a control group of mice;
administering a drug to the treatment group;
administering a vehicle to the control group; and
measuring tumor volume of the treatment group and tumor volume of the control group at a plurality of days;

determining progress-free survival (PFS) or overall survival (OS) of the treatment group and the control group;
evaluating efficacy of the drug based on the PFS or OS using an additive frailty model.

In certain embodiments, the additive frailty model includes a hazard function, wherein the hazard function for the *j*-th mouse of the i-th PDX is

$$h_{ij}(t) = h_0(t)\exp(u_i + (w + v_i)T_{ij} + \beta^{\mathrm{T}}X_i)$$

wherein $h_0(t)$ is a baseline hazard function, $u_i$ is a random effect associated with the *i*-th patient without drug treatment, $w$ is mean drug effect, $v_i$ is a random effect associated the *i*-th patient that depicts drug response deviating from $w$, $T_{ij}$ is 0 for the control group and 1 for the treatment group, $X_i$ is a vector for a covariate of the plurality of patients, $\beta^{\mathrm{T}}$ is a vector quantifying fixed effects of the covariate.

In certain embodiments, the PFS is the time until the tumor volume doubles.

In certain embodiments, the covariate is cancer type, expression of a gene or a gene mutation.

In certain embodiments, the treatment group consists of n mice for each patient and the control group consists of n mice for each patient, wherein n ≥ 3.

In certain embodiments, the method disclosed herein further comprises conducting a second mouse clinical trial based on the hazard function.

As used herein, the term "gene" refers broadly to any nucleic acid associated with a biological function. Genes typically include coding sequences and/or the regulatory sequences required for expression of such coding sequences. The term gene can apply to a specific genomic sequence, as well as to a cDNA or an mRNA encoded by that genomic sequence. "Gene expression" refers to the process by which information from a gene is used in the synthesis of a functional product, including protein and functional RNA (e.g., tRNA, snRNA, and microRNA). In certain embodiments, the expression level of a gene can be measured by the transcript (e.g. mRNA) of the gene or the derivative thereof (e.g. cDNA).

In certain embodiments, N is between 20 and 80. In certain embodiments, N is about 30, 40, 50, 60, 70, 80, 90 or 100. In certain embodiments, N is around 50.

In certain embodiments, the gene expression profile described herein is obtained by transcriptome RNA sequencing or microarray. In certain embodiments, the gene expression profile described herein is obtained from the cancer genome atlas (TCGA) dataset.

In certain embodiments, the method described herein is computer-implemented, i.e., the method is carried out in a computer, e.g., a computer program executed by a CPU. A computer, as used herein, refers to a device (for general or specific purposes) that can be programmed to perform a set of arithmetic or logical operations automatically. Computers, as used herein, include without limitation personal computers, workstations, servers, mainframes and supercomputers. The computer can be a stand-alone system, networked system or a virtual machine residing in a computing cloud. The methods described herein can be implemented with multithreading or other parallel computing methods.

The following examples are presented to illustrate the present invention. They are not intended to limiting in any manner.

EXAMPLE 1

This example shows that xenograft tumor growth largely follows exponential kinetics.

With an aim at developing new method of preclinical cancer pharmacology, we first set out to examine the growth patterns of xenograft tumors. Tumor grows by doubling of tumor cells, therefore, should follow exponential growth in theory. We thus began by testing actual PDX growth to see if it indeed follows exponential kinetics using our large pharmacology datasets of PDX trials. The exponential growth kinetics can be described by

$$V_x = V_0 e^{kx} \qquad (1)$$

where $V_0$ is the initial tumor volume normally ranging from 50-300mm$^3$, $V_x$ is the tumor volume at day $x$, $k$ is a positive rate parameter. A logarithmic transformation on both sides of the above equation gives the linear equation

$$\ln V_x = \ln(V_0) + kx \qquad (2)$$

by which the estimate of $k$, termed $\hat{k}$, is obtained. The tumor volume doubling time (T$_2$) is then computed by $\frac{\ln(2)}{\hat{k}}$. Faster growing tumors have larger $k$ and smaller T$_2$.

We applied Equation 2, by ordinary least square regression, to model tumor growth for 6310 PDX mice under vehicle treatment and had data for at least 5 days. Tumor volumes were usually measured every 3 to 4 days (twice weekly) before reaching 3000mm$^3$. We found that the coefficient of determination (R$^2$) is >0.70 for nearly 100%, and >0.90 for 90% of the mice.

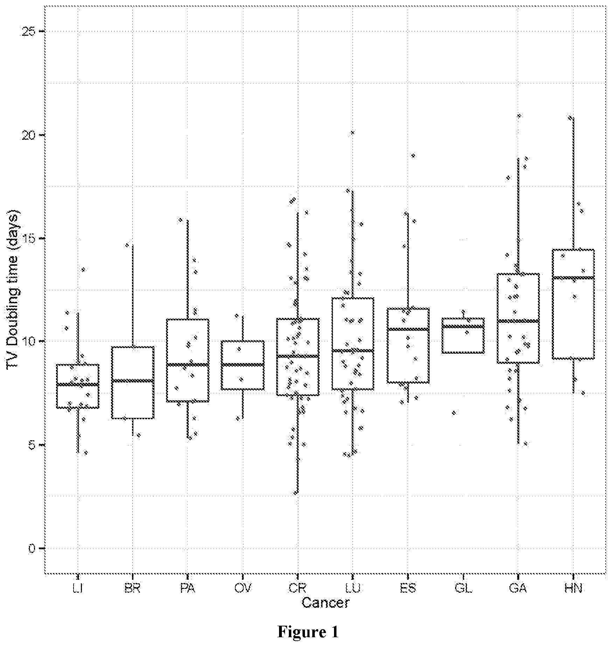

Tumor growth rates were found to vary within and between cancer types (**Figure 1**). The median T$_2$ ranges from 8.0 days in liver cancer and breast cancer to 13.1 days in head and neck cancer, and significant difference is seen across the 10 cancer types with at least 4 PDXs (one-way ANOVA p-value = 0.002). Within a cancer type, T$_2$ ranges from about 2-fold in gallbladder cancers and ovarian cancers to about 6-fold in colorectal cancers and lung cancers. The average T$_2$ across all PDXs is 9.8 $\pm$ 4.8 days.

Tumor growth rate also varies among the mice within a PDX model. The coefficient of variation for T$_2$ is 0.29 $\pm$ 0.12 (mean $\pm$ SD), ranging from to 0.07 to 0.74, for PDXs with data for at least 5 mice. The coefficient of variation reduces to 0.20 $\pm$ 0.09, ranging from 0.03 to 0.74, when calculated by individual projects, under which mice of a PDX were implanted with segments of the same tumor chunk, treated and monitored in the same experimental condition. Thus, a significant portion of the growth variation should stem from tumor heterogeneity. In summary, under vehicle treatment, most PDX tumors exhibit exponential growth until tumor volume reaches between 2000-3000mm$^3$.

EXAMPLE 2

This example illustrates drug treatment causes complex growth kinetics in PDXs.

We next examined tumor growth kinetics under various drug treatments. We examined growths of 27771 PDX mice, and found that tumor shrunk for at least one measurement day in 26.0% of them, though some might result from measurement error. At termination point, tumor volume ratio (V$_e$/V$_0$) of end tumor volume (V$_e$) versus dosing starting volume (V$_0$) are found to be 0% (2.0% of mice), < 100% (10.8% of the mice), -100% (-5% of the mice), < 173% (19.8% of mice), < 200% (22.8% of mice). Studies also ran longer in drug groups (29.7 $\pm$ 14.8 days) than in vehicle groups (24.0 $\pm$9.1 days).

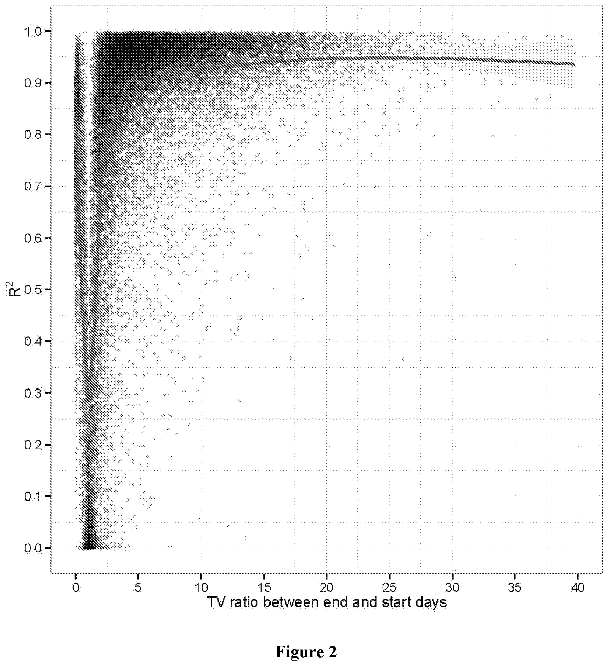

We then attempted to assess whether tumor growth still follows exponential kinetics under drug treatment. In general, a more potent drug led to smaller tumors at the end of a study. There was a relationship between the regression R$^2$ and V$_e$/V$_0$, the ratio between the ending and starting tumor volumes (**Figure 2**). The average R$^2$ was smallest when V$_g$/V$_0$ was -1, as expected mathematically, and R$^2$ increased quickly when V$_e$/V$_0$ went down and up. The percentage of growth curves with R$^2$ > 0.70 was 91.8%, 95.1%, and 96.3% when V$_e$/V$_0$ was larger than 2, 3, and 4, respectively. If V$_e$/V$_0$ < 0.5, about 70% of growth curves had R$^2$ >0.70. Therefore, under drug treatment, most tumors either did not grow (or shrink) much, or grew (or shrink) exponentially that can be well modeled by Equations (1) and (2).

Overall, around 20% of growth curves under drug treatment were not exponential, as suggested by a clustering analysis (**Figure 3A**). Indeed, when Equation 2 was used to model tumor growth, we found that R$^2$ is >0.70 for 80% of the growth curves. However, only about 8% of tumors had non-exponential growth curves with V$_e$/V$_0$ >2 or <0.5 (**Figure 3B**). Among them, 10-20% had irregular shapes, the remaining had V-like and $\Lambda$-like shapes. The former might suggest development of drug resistance and the latter a latent period before drug effect took place. Overall, tumor growth under treatments displayed more complex kinetic patterns as compared to no treatment.

EXAMPLE 3

This example illustrates that TGI is a tumor response endpoint biased by tumor growth rate and time of study termination.

We next investigated Tumor Growth Inhibition (TGI), a commonly used tumor response endpoint, and its variants or equivalent end points, to determine its adequacy for pharmacology evaluation. TGI is defined as

$$TGI = 1 - \frac{\Delta T}{\Delta C} = 1 - \frac{V_x^{(T)} - V_0^{(T)}}{V_x^{(C)} - V_0^{(C)}} \qquad (3)$$

where $V_x^{(T)}$ and $V_0^{(T)}$ are the tumor volume at day $x$ and day 0 in the drug treatment group; $V_x^{(C)}$ and $V_0^{(C)}$ are the corresponding volumes in the vehicle or control group. TGI and $\Delta T/\Delta C$ equivalently used in practice, and frequently presented in percentage. Usually, there are multiple mice in both drug and vehicle groups, so these 4 tumor volume parameters are the averages or medians. By definition, if there is no response, then TGI=0. If a drug causes tumor growing faster than vehicle, then TGI<0. If tumor growth is inhibited by treatment, then TGI>0; if the tumor volume does not change, then TGI=1, and if the tumor shrinks, i.e. $0 \leq V_x^{(T)} < V_0^{(T)}$, then TGI >1.

To assess the time-dependency of TGI, we assumed that tumor grows exponentially in both drug and vehicle groups with rate constants $k_T$ and $k_C$, respectively, and with equal starting volumes, i.e. $V_0^{(T)} = V_0^{(C)}$, then

$$TGI = 1 - \frac{e^{k_T x} - 1}{e^{k_C x} - 1} \qquad (4)$$

In our PDX data, $k_C$ ranges from 0.0139 to 0.307, and $k_T$ ranges from -0.488 to 0.273. The rate ratio $k_T/k_C$ is a constant that measures drug response, if both groups grow exponentially. For example, if $k_T/k_C$ = 0.5, then a drug inhibits tumor growth by half, so that it takes twice the time to double the tumor volume under drug than vehicle treatment. The smaller the ratio is, the more potent the drug is. A negative $k_T/k_C$ value indicates tumor shrinkage under drug treatment.

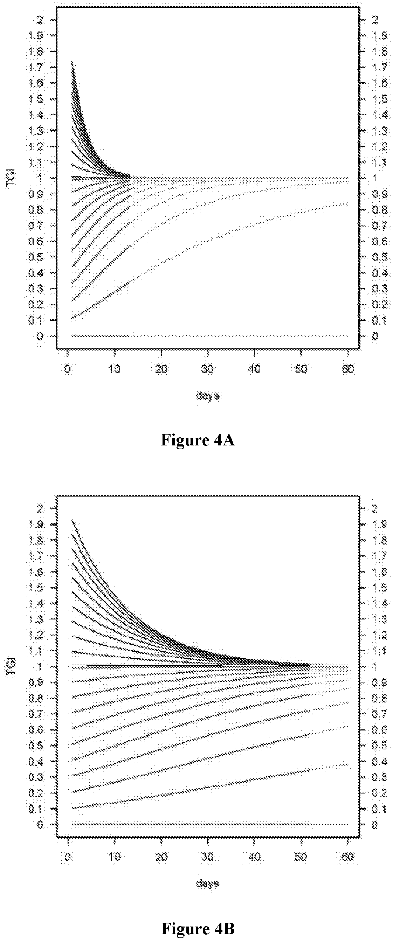

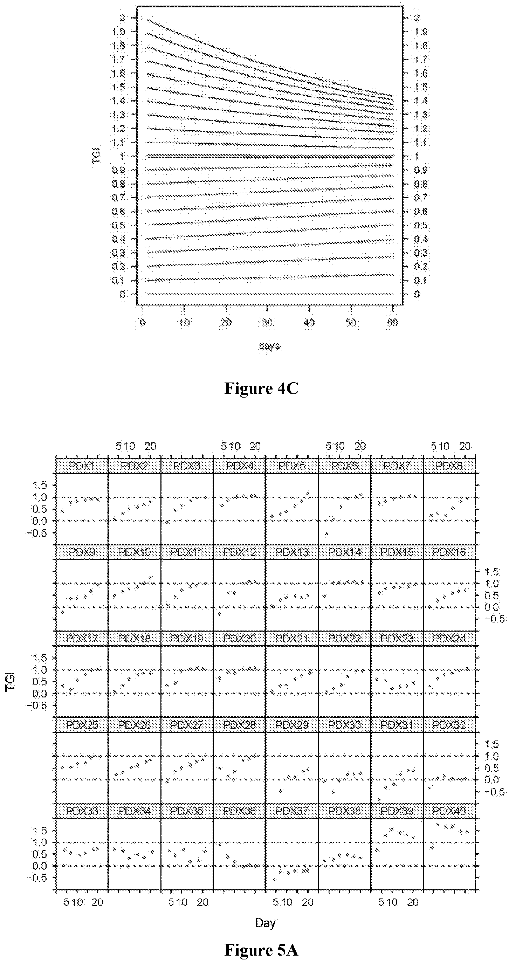

Drug should almost always inhibit tumor growth, therefore, $k_T < k_C$ and TGI>0. We plotted TGI values against days-post initial dosing for the first 60 days, when 99.5% of our studies ended (Figure 4A-C). With $k_T/k_C$ as a tumor response measurement, we observed: 1) TGI is time-dependent for any given $k_T/k_C$ value. If TGI>1, its value goes down toward 1 with time; if 1>TGI>0, its value goes up toward 1 with time, even more so for faster growing tumors; 2) TGI is dependent on tumor growth rate ($k_C$). For a given $k_T/k_C$, TGI at a fixed time was larger for faster growing (larger $k_C$) tumors if between 0 and 1, and smaller if >1.

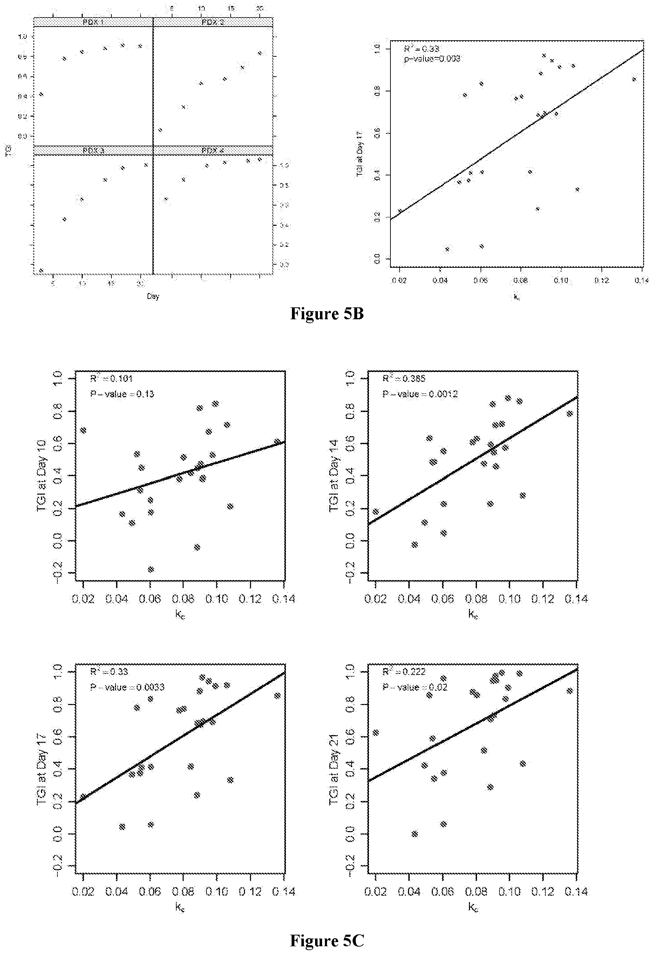

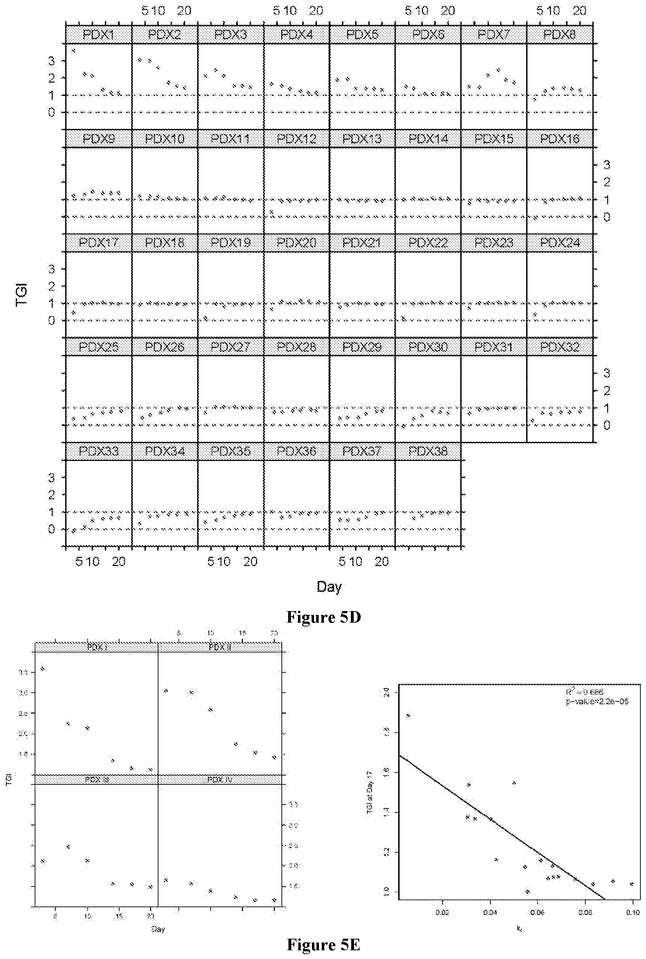

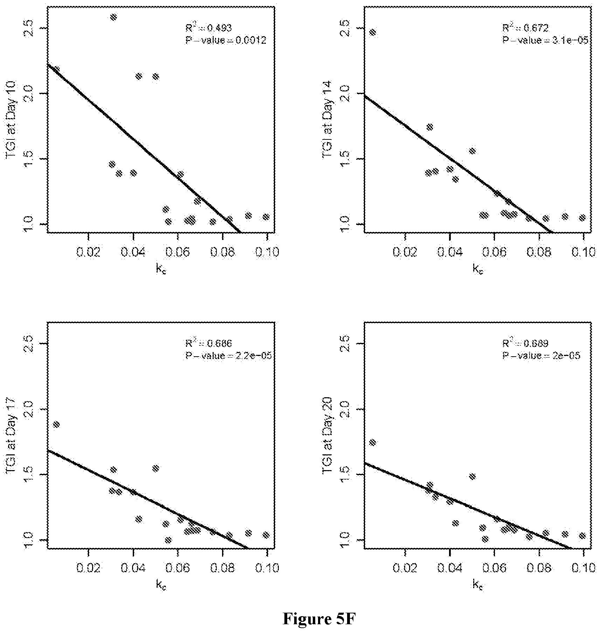

We then tested the above theoretical predictions by examining a MCT dataset consisted of 40 PDXs with 3:3 design format (3 mice in treatment/3 in vehicle groups for each PDX). Tumor volumes were measured at day 0, 3, 7, 10, 14, 17, and 21. The average starting tumor volumes $V_0^{(T)}$ and $V_0^{(C)}$ were between 115mm³ and 194mm³, and for a PDX, $V_0^{(T)}$ and $V_0^{(C)}$ differ by 4.7% on average. We found that the first two TGIs at day 3 and 7 frequently behave irregularly, likely caused by the initial unstable response to drug treatment and the relatively large measurement errors when tumors are small (**Figure 5A**). Thereafter, TGIs varied with time as expected in about 90% PDXs. The drug was very potent, 39 PDXs showed certain level of response, and tumor shrunk in 2 PDXs. We found strong positive correlations between $k_C$ and TGIs at day 14, 17 and 21 (Figure 5B-D) for 24 PDXs with all TGIs between 0 and 1. The correlation at day 21 became weaker because TGIs for many PDX were getting close to 1. In another MCT dataset of 38 PDXs, we observed strong negative correlations between $k_C$ and TGIs for 18 PDXs 4 measurement days when TGI is larger than 1 (**Figure 5E-5F**).

TGI as tumor response readout may have other drawbacks. First, in the above analysis, we stipulated $V_0^{(T)} = V_0^{(C)}$, or that tumors are in the same growth phase when dosing starts. In practice, however, it is not always possible to ensure such matching, especially when the number of mice for a PDX is small, which could affect TGI values dramatically. For example, if $V_0^{(T)} = \frac{1}{2} V_0^{(C)}$, then a TGI originally being 0.5 becomes 0.75. Secondly, tumor volumes, consequently TGIs, may fluctuate irregularly from day to day, especially in drug treatment groups. It is difficult to determine which day's TGI should be used. Thirdly, it is a common practice to use TGI from the same day for comparison, however, it may not be available for some fast growing tumors. These inflexibilities of TGI, in addition to its inherent inaccuracy, may limit its application as a tumor response readout.

There are some alternative definitions of TGI, notably $TGI = 1 - \frac{V_x^{(T)}}{V_x^{(C)}}$ and $TGI = 1 - \frac{V_x^{(T)}/V_0^{(T)}}{V_x^{(C)}/V_0^{(C)}}$. The

terms $\frac{V_x^{(T)}}{V_0^{(T)}}$ and $\frac{V_x^{(C)}}{V_0^{(C)}}$ are the relative tumor volumes. Assuming tumor grows exponentially in both drug and vehicle groups, these two definitions reduce to $TGI = 1 - \frac{V_0^{(T)} e^{k_T x}}{V_0^{(C)} e^{k_C x}}$ and $TGI = 1 - \frac{e^{k_T x}}{e^{k_C x}}$, which become equivalent if

$V_0^{(T)} = V_0^{(C)}$. We found that they were more sensitive in their dependence on time and growth rate than TGI defined in Equation 3 (Figure 6A-C).

EXAMPLE 4

This example illustrates that AUC ratio is an unbiased metric based on $k_T/k_C$ measuring tumor response with superiority to TGI

We mentioned in EXAMPLE 3 that $k_T/k_C$ is a constant measuring tumor response under the exponential growth assumption. By Equation 2, it is easy to show that

$$\frac{k_T}{k_c} = \frac{\int_0^d \ln V_x^{(T)} dx - d \times \ln V_0^{(T)}}{\int_0^d \ln V_x^{(C)} dx - d \times \ln V_0^{(C)}} = \frac{AUC_T}{AUC_C} \qquad (5)$$

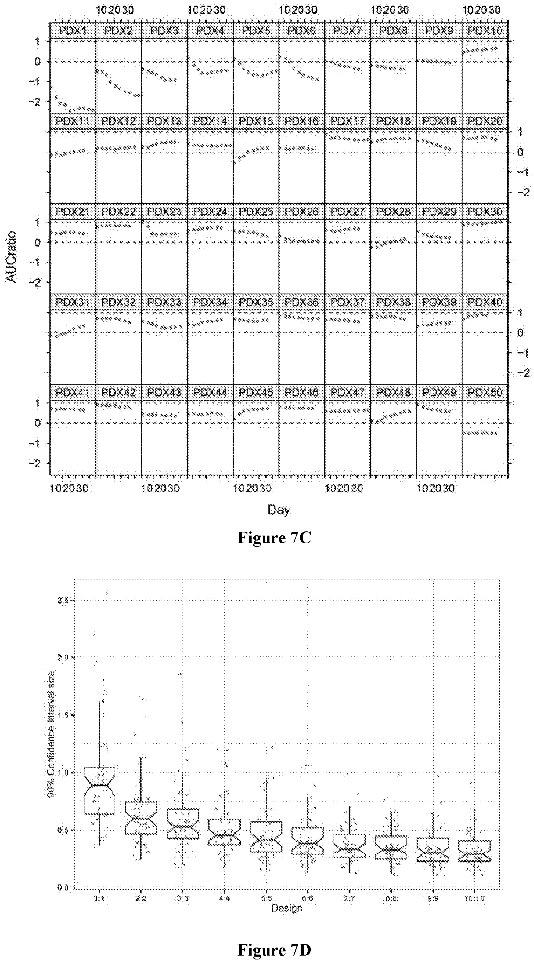

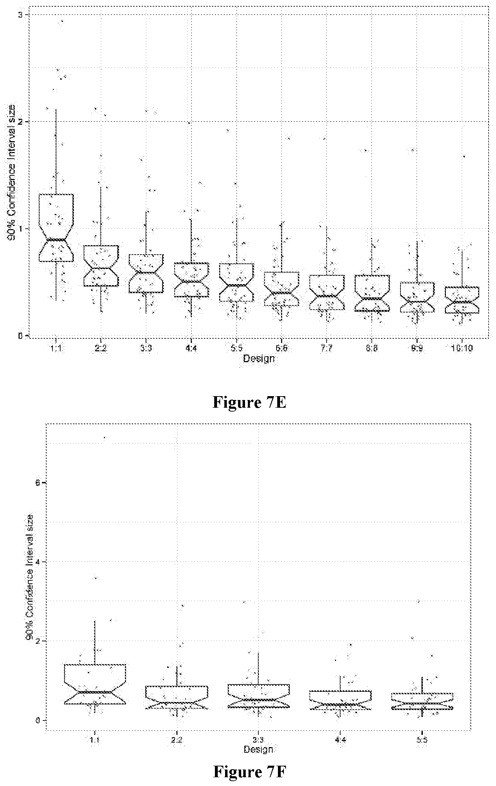

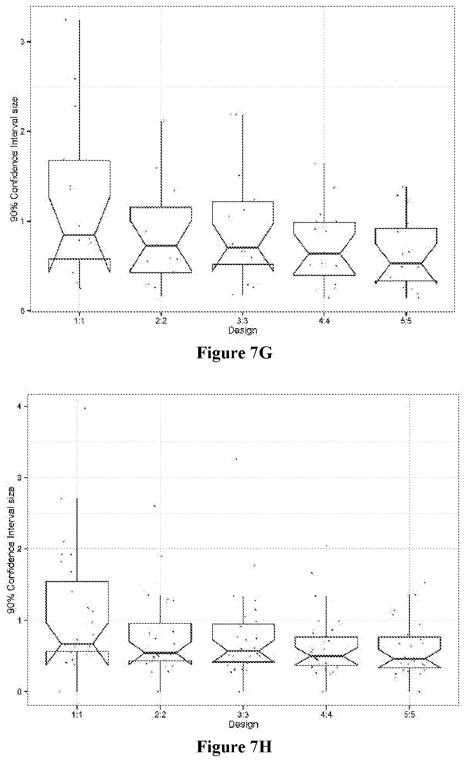

The two starting tumor volumes, $V_0^{(T)}$ and $V_0^{(C)}$, need not to be the same. These two AUCs (area under curve) are illustrated in (**Figure 7A**), and the AUC ratio equals $k_T/k_C$. If a study stops at different time for the two groups, $d_T$ for the drug group and $d_C$ for the vehicle group, we need to normalize the AUCs by $d_T^2$ or $d_C^2$. For convenience, we still call the normalized AUC as AUC, and the ratio between the two normalized AUCs as the AUC ratio:

$$\frac{k_T}{k_c} = \frac{AUC_T/d_T^2}{AUC_C/d_C^2} \qquad (6)$$

A smaller AUC ratio indicates better drug efficacy. When the ratio is negative, tumor shrinks under drug treatment; when the ratio is larger than 1, tumor grows faster under drug treatment.

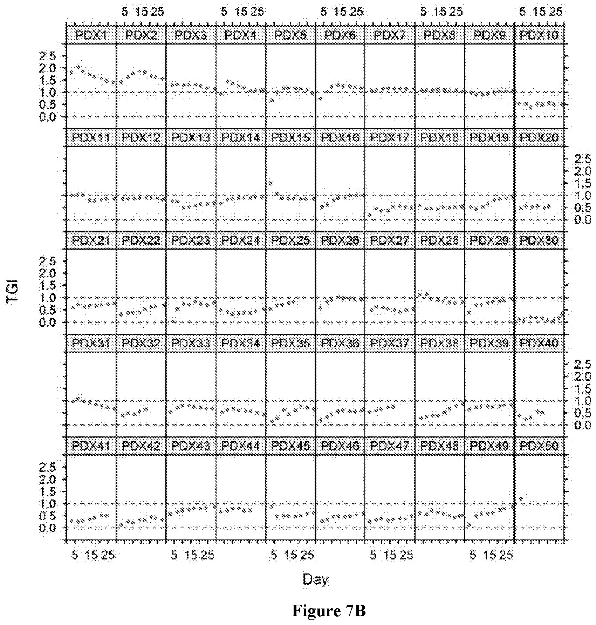

We can use the trapezoidal rule to obtain AUCs from the growth curves, even when they are non-exponential, for which AUC is a measure of average drug effect during study period. Similar to TGI, AUC ratio varies more in the first week(s) before reaching stable response. Unlike TGI that approaches 1 with time, AUC ratio converges to a value, which is $k_T/k_C$ under exponential growth kinetics, and which may be far away from 1 (**Figure 7B-7C**).

For multiple mice in drug group ($n_T$) and vehicle group ($n_C$), AUCs in each group can be viewed as random samplings from corresponding normal distributions with sizeable standard deviations against the corresponding mean. The two normal distributions can differ in both mean and standard deviation. AUC ratio, therefore, follows a Cauchy-like distribution with heavy tails (Hinkley DV, Biometrika (1969) 56(3):635-39), where the median of ratios is a better metric than the ratio of medians (Brody JP et al., PNAS (2002) 99(20): 12975-8). We computed the $n_T \times n_C$ AUC ratios, then take their median to calculate the median of ratios.

To calculate the ratio of medians, we first computed the median of the $n_T$ AUCs and the median of the $n_C$ AUCs, then took their ratio. All AUC ratios in the tables and graphs, unless specified otherwise, were obtained by the median-of-ratios method.

We used a bootstrap procedure to obtain the confidence interval (CI) of AUC ratio. Specifically, we performed a random sampling with replacement to get $n_T$ AUCs from drug group and $n_C$ AUCs from vehicle group, then calculated median AUC ratio. This procedure was repeated 1000 times to get a list of median AUC ratios, from which an equitailed 90% confidence intervals was constructed. The distribution of the median AUC ratio was often non-normal and skewed due to small number of mice and diverse drug response, under which the bootstrap confidence intervals were usually much tighter than ones calculated by formulas based on normal distribution, e.g., $\overline{x} \pm 1.96 SE(\overline{x})$ for the 95% confidence interval. The bootstrap confidence interval got smaller when mice had more similar tumor growth curves.

We further investigated how number of mice affects estimate error of the median AUC ratio. For simplicity, we assumed a balanced design with equal number of mice in the two groups (n:n design), and we estimated the bootstrap CI widths. From a MCT study with 50 PDXs and with 10 mice each in vehicle group and drug group, we calculated the 90% bootstrap confidence interval of the AUC ratios, and we found that the 1:1 design has very large CI width. Having more than one mouse per PDX model substantially reduced the CI width. 3:3 and up designs appeared to be a reasonable balance of measurement accuracy and cost for this specific case (**Figure 7D**). Similar observations were seen in several other MCTs (Figure 7 E-H).

Our results show that the AUC ratio gives great flexibility for studies: mice can be enrolled as long as tumor volumes are between 100-300mm$^3$, and there is no need to match starting tumor volumes between a drug group and a vehicle group. Studies can be stopped at different time for different PDXs, suited to their tumor growth rates, and drug efficacy is calculated using tumor volumes from nearly any time. If a drug has good efficacy, the drug group can run longer than the vehicle group to get better characterization of its efficacy and response profile, besides getting more accurate AUC ratio. Mice for a PDX can have very different growth rates, in a drug or in vehicle group, which can all be captured by individual AUCs. In summary, AUC ratio displays significant operational robustness, in addition to accuracy, as compared to TGI.

EXAMPLE 5

This example illustrates methods for MCT data analysis using the AUC ratio.

A MCT, similar to a phase-2 human clinical trial, is a population based investigation where tumor response rate (RR) and survival parameters are two standard efficacy readouts. Unlike human studies, a MCT can have multiple arms (vehicle, comparator arms, dosages, etc.) in which a given PDX can present simultaneously and each PDX model maybe represented by multiple mice. In other words, conceptually, a cancer patient simultaneously participates multiple arms and has replicates in each arm. This makes MCT a powerful preclinical tool. However, there is a need for new methods to support MCT data analysis that are different from those for individual preclinical studies and also those for human trials. The following describes cases assessing the correlation of EGFR gene expression to gastric PDX response to cetuximab using the AUC ratio.

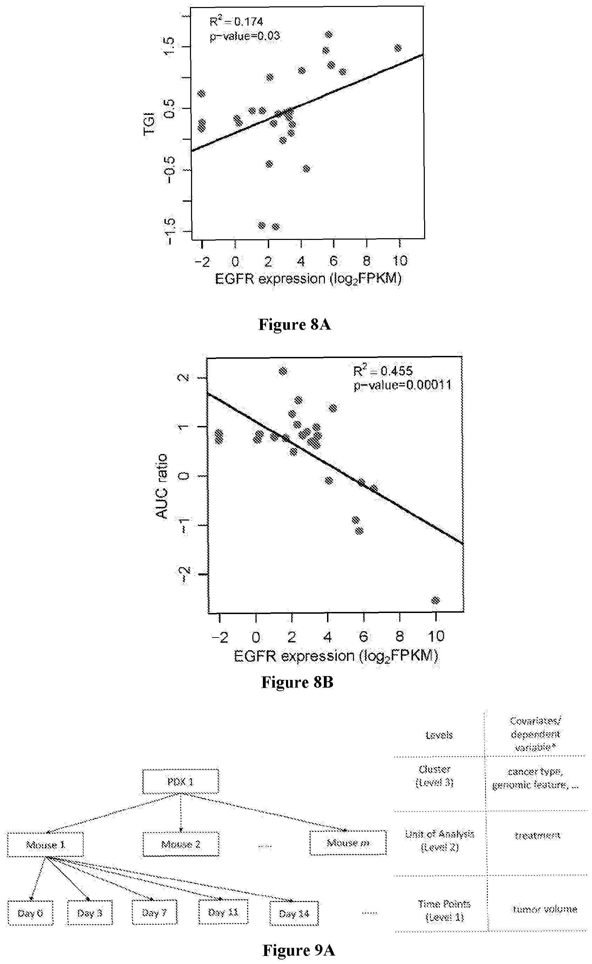

We previously described a MCT study involving a cohort of 20 gastric PDXs where EGFR overexpression seems associated with positive response to cetuximab PDXs based on TGI type of response endpoint (Zhang L et al., Scientific Reports (2013) 3:2992). We expanded the studies to 27 PDXs, completed at the different times with other study variations: mouse numbers per arm (3-10 in a drug or vehicle group), durations (14-63 days), *etc*. We calculated TGIs at day 14, and also AUC ratios using full set of data for all 27 PDXs. We observed a much stronger correlation between EGFR mRNA levels and the AUC ratios ($R^2$=0.45, p-value=1.1 $\times$ 10$^{-4}$, **Figure 8A**), than between EGFR level and TGIs ($R^2$=0.17, p-value=0.03, **Figure 8B**). The most responsive GA0152 had extremely high EGFR expression (FPKM>1000). Furthermore, for the 7 PDXs with high EGFR levels (FPKM around or larger than 20), 6/7 experienced tumor volume reduction under treatment, with only exception of GA2140 that was the third most resistant. GA2140 carries EGFR L435R (Ensemble ENST00000455089) missense mutation, predicted to be disruptive to protein function by SIFT(37), and might be a biomarker for cetuximab resistance. This analysis also supports that AUC is more robust tumor response readout over TGI in MCT and particularly for biomarker discovery. Our observation that EGFR over-expression is a predictive biomarker to cetuximab is supported by a phase 2 trial and a phase 3 trial with data reinterpretation (Zhang X et al., Medical Oncology (2014) 31(10):226). Specifically, we found that for patient with IHC score greater than -200, the 7 patients receiving cetuximab in addition had significantly longer PFS (8.1 $\pm$ 6.1 months, one-sided Mann-Whitney U-test p-value=0.03) and OS (19.5 $\pm$ 12.0 months, p-value=0.02) than the 19 patients receiving only chemotherapies (PFS= 4.1 $\pm$ 3.2 months, OS=10.1 $\pm$ 7.8 months). Two censored patients with very low OS and PFS in the chemotherapy group were excluded from the analysis.

EXAMPLE 6

This example illustrates methods for MCT data analysis using linear mixed models.

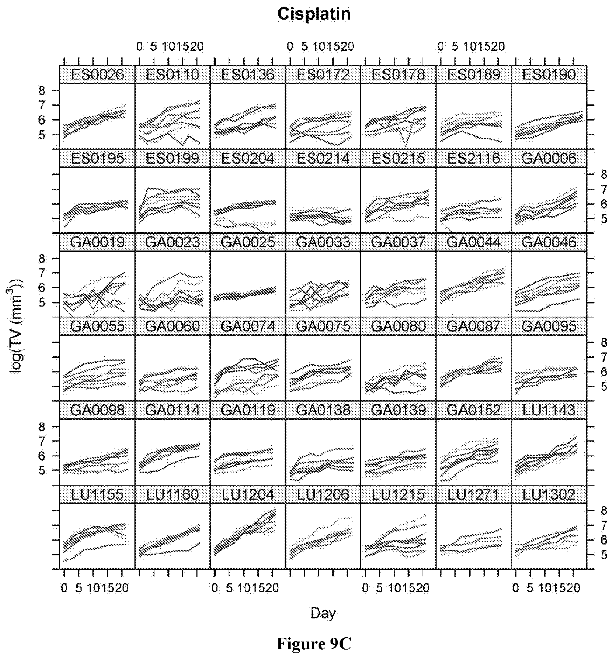

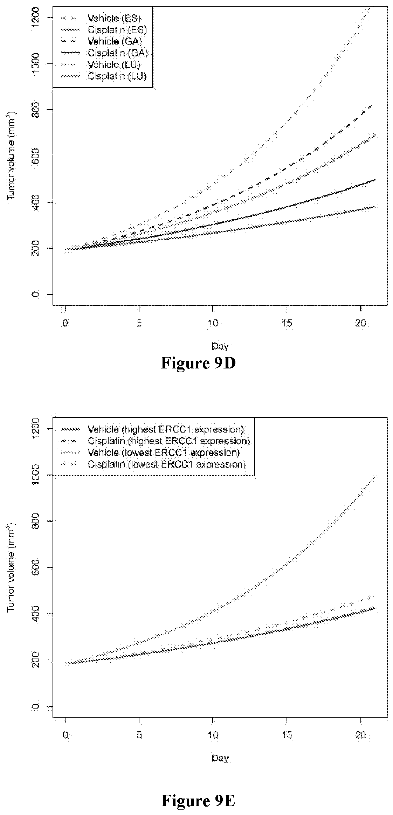

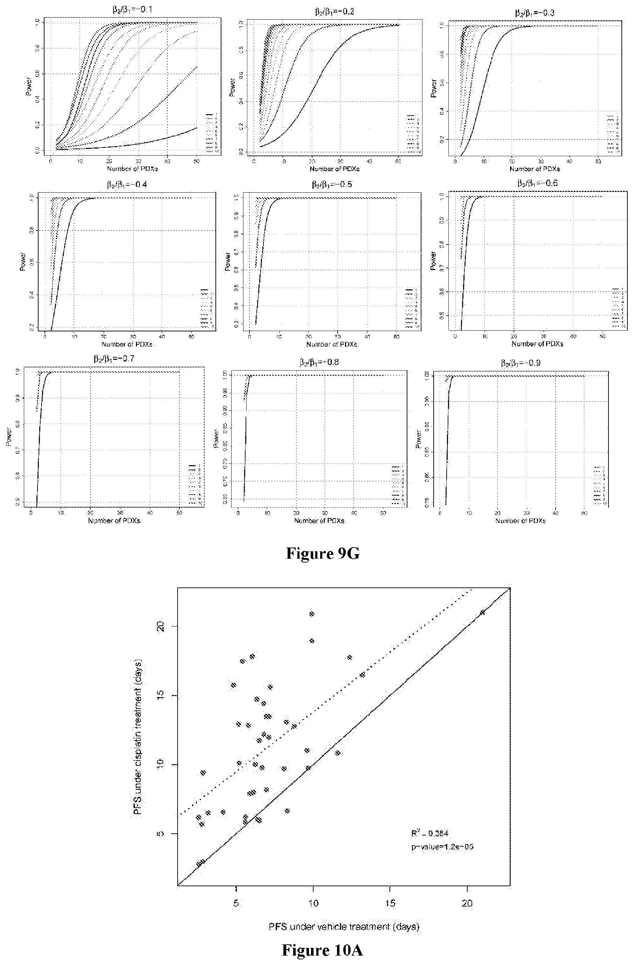

In a MCT, tumor volume is measured at multiple time points for each mouse, and these tumor volumes are often correlated, as in a typical longitudinal study. As shown above, almost all growth curves under vehicle treatment can be modeled by the linear Equation 2, and under drug treatment, about 80% of the growth curves are linear or close to linear. If we introduce a quadratic term on the day ($x^2$), more than 90% of the growth curves can be fitted with $R^2$>0.7, and 97% with $R^2$>0.4. With such strong linearity, the linear mixed models (LMM) is a proper and powerful tool to analyze the clustered longitudinal data from MCTs(West BT et al., "Linear Mixed Models: A Practical Guide Using Statistical Software, Second Edition", Crc Press (2014)). Specifically, a 3-level model is appropriate, in which PDX and mouse are random factors, and covariates at the PDX-level and mouse-level can be included (**Figure 9A**). We used an MCT to illustrate the application of LMMs for drug response evaluation and biomarker discovery.

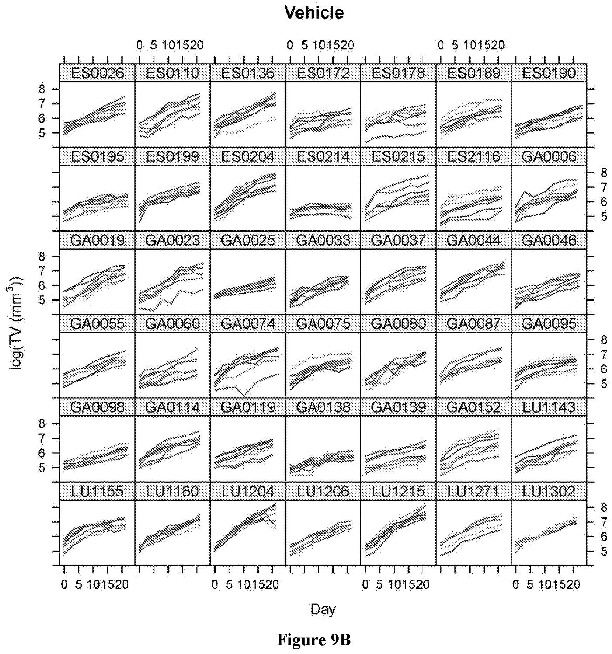

The MCT evaluated cisplatin response (4mg/kg, daily dosing for 3 weeks) on 42 PDXs including 13 esophageal cancers (ES), 21 gastric cancers (GA) and 8 lung cancers(LU), each PDX had 5-9 mice (Figure 9B-C). A general model was specified for tumor volume, in log scale, at day $t$ for mouse $i$ within PDX $j$ as follows:

$$\ln V_{tij} = \beta_0 + \beta_1 \times Day_t + \beta_2 \times Day_t \times CancerTypeGA_j + \beta_3 \times Day_t \times CancerTypeLU_j + \beta_4 \times Day_t \times Treatment_{ij} + \beta_5$$
$$\times Day_t \times CancerTypeGA_j \times Treatment_{ij} + \beta_6 \times Day_t \times CancerTypeLU_j \times Treatment_{ij} + u_{0j} + u_{1j}$$
$$\times Day_t + u_{(0i|j)} + u_{(1i|j)} \times Day_t + \varepsilon_{tij}$$

$$(7)$$

The model used vehicle in ES as the reference. There were 6 fixed effects, $\beta_0$ for the intercept, $\beta_1$ for the time slope, $\beta_2$ and $\beta_3$ quantify the growth rate difference of GA and LU with respect to ES, $\beta_4$ measures cisplatin effect, $\beta_5$ and $\beta_6$ check if GA and LU respond differently to cisplatin. The model also had 5 random effects, including the residual $\varepsilon_{tij}$. In a MCT, we can view the cohort of PDXs as random samples from a PDX or patient population, therefore, they have different growth rates, which is modeled by the random effect $u_{1j}$ associated with the time slope. Similarly, we model the growth difference for mice within a PDX by the random effect $u_{1i|j}$. Mice and PDX may also have different starting tumor volumes, which are modeled by the two random effects on intercept: $u_{0j}$ and $u_{0i|j}$. Therefore, our LMM captures both PDX-level and mouse-level growth heterogeneity under vehicle and drug treatments. From the modeling fitting (**Table 1**), we observe that (1) tumor in GA grows slightly faster than ES, while tumor growth is much faster in LU than in ES and GA; (2) cisplatin caused comparable responses on the 3 cancer types (p-values for $\beta_5$ and $\beta_6$ are >0.05). From the model, we can get fitted cancer specific growth curves, as well as PDX and mouse specific growth curves. On average, cisplatin reduces tumor growth rate by about 38%. The AUC ratio method gives a close 36%.

**Table 1**. Parameters estimated for the LMM (Equation 7) of the cisplatin dataset

| Fixed-Effect Parameters | Estimate* | p-value |
|---|---|---|
| $\beta_0$ (Intercept) | 5.2641(0.0257) | 0 |
| $\beta_1$ (Day) | 0.0605(0.0043) | 1.5E-43 |
| $\beta_2$ (Day $\times$ CancerTypeGA) | 0.0091(0.0055) | 0.098 |
| $\beta_3$ (Day $\times$ CancerTypeLU) | 0.0297(0.0071) | 2.8E-5 |
| $\beta_4$ (Day $\times$ Treatment) | -0.0282(0.0031) | 1.2E-19 |
| $\beta_5$ (Day $\times$ CancerTypeGA $\times$ Treatment) | 0.0037(0.0039) | 0.35 |
| $\beta_5$ (Day $\times$ CancerTypeLU $\times$ Treatment) | -0.0011(0.0052) | 0.84 |
| *parameters estimated by the REML method in the R nlme package. | | |

The LMM can readily incorporate PDX-level genomic covariates, e.g. gene expression and mutation, for biomarker analysis. We used the same dataset to examine if ERCC1 expression is related to cisplatin response in gastric cancer by fitting the following LMM:

$$\ln V_{tij} = \beta_0 + \beta_1 \times Day_t + \beta_2 \times Day_t \times ERCC1_j + \beta_3 \times Day_t \times Treatment_{ij} + \beta_4 \times$$
$$Day_t \times ERCC1_j \times Treatment_{ij} + u_{0j} + u_{1j} \times Day_t + u_{(0i|j)} + u_{(1i|j)} \times Day_t + \varepsilon_{tij}$$

$$(8)$$

In this model, $\beta_2$ evaluates ERCC1 expression as a prognostic biomarker since it is for both vehicle and cisplatin groups. The fitted value of $\beta_2$ is -0.0155, indicating that higher expression of ERCC1 leads to slower tumor growth (**Figure 9E**). The coefficient $\beta_4$ examines ERCC1 expression as a predictive biomarker for cisplatin. The fitted value of $\beta_4$ is 0.0136, close to that of $\beta_2$. Therefore, high ERCC1 expression acts adversely to cisplatin treatment (**Figure 9E**). The parameter $\beta_3$ quantifies cisplatin treatment and is estimated to be -0.0879. Results from our LMM may have clinical implications.

Our results indicates that patients with lower ERCC1 expression are likely to have worse tumor progression but also benefit more from cisplatin and likely other platinum-based chemotherapies. These two effects work against each other and might be in comparable magnitude. Accordingly, it is hard to evaluate ERCC1 expression as a predictive biomarker in relevant clinical trials when PFS and OS are used as end points. Indeed, controversial trial conclusions exist. While more trials claim that patients benefit from low ERCC1 expression (Metzger R et al., J Clinical Oncology (1998;) 16(1):309-16; Kwon HC et al., Annals of Oncology (2007) 18(3):504-9; Hirakawa M et al., Cancer Chemotherapy and Pharmacology (2013) 71(3):789-97; Miura JT et al., Cancer Biology & Therapy (2015) 16(5):764-9), there are some stating that overexpression of ERCC1 is related to longer survival (Bamias A et al., Cancer Chemotherapy & Pharmacology (2010) 65(6):1009-21; Baek SK et al., Cancer Research & Treatment (2006) 38(1):19-24; Kim KH et al., Biomarkers (2011) 16(1):74-82). Still others found no connection (Sonnenblick A et al., Medical Oncology (2012) 29(5):3035-8). In one study, the researchers found that high ERCC1 expression is associated with decreased OS in chemotherapy but with increased OS in patients with surgery alone (Squires MH et al., Cancer (2013) 119(17):3242-50). Our MCT supports the notation that patients with low ERCC1 benefit more from cisplatin chemotherapy.

The LMM uses all tumor volume data, even if some mice may have less data points than others. We performed a power analysis based on parameters estimated from fitting the cisplatin dataset by a simple LMM:

$$\ln V_{tij} = \beta_0 + \beta_1 \times Day_t + \beta_2 \times Day_t \times Treatment_{ij} + u_{0j} + u_{1j} \times Day_t + u_{(0i|j)}$$
$$+ u_{(1i|j)} \times Day_t + \varepsilon_{tij}$$

$$(9)$$

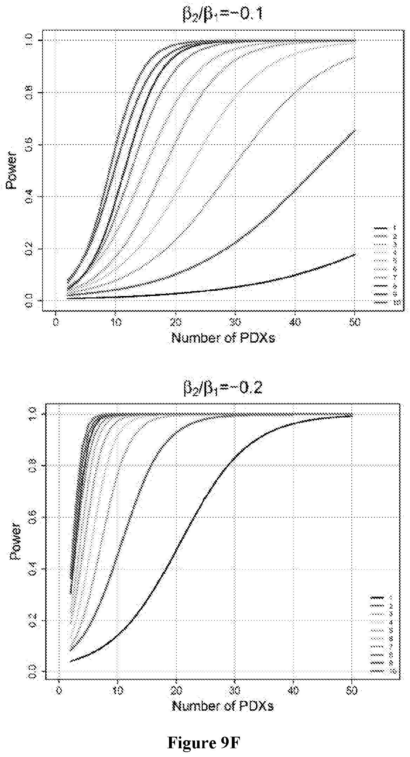

We assumed n:n designs so that for each PDX, there were n mice in the vehicle group and *n* mice in the drug group. The MCT run for 3 weeks, and all mice had tumor volume data for 7 measurement days including day 0. At significance level a=0.05, we obtained power curves by simulations for $\beta_1/\beta_2$= -0.1 to -0.9, that is, the drug treatment reduces tumor growth rate by 10% to 90% (**Figure 9F-9G**). We observed 1) the 1:1 design had much weaker power than any design with more than one mouse per PDX (n > 1); and 2) power increased quickly from 1: 1 design to 2:2 design, and from 2:2 design to 3:3 design, after that, the increase slowed down significantly. Therefore, having 3 or more mice per PDX per treatment can give MCTs adequate power with a much smaller number of PDXs.

Next, we attempted to use power curves to guide MCT design. We set to perform a hypothetic MCT on multiple cancer types to prioritize indications for future human trials. We stipulated a 20% efficacy difference as the selection threshold. Per **Figure 9E,** we can conduct MCT by one of the following enrolling schemes to achieve 0.05 significance level and 90% power: 1) 35 PDXs for each cancer type and each PDX with one mouse; 2) 20 PDXs for each cancer type and each PDX with 2 mice; 3) 13 PDXs each with 3 mice, 4) 10 PDXs for each cancer type and each PDX with 4 mice, and so on. This gives flexibility in operations, especially when the availability of PDXs and mice is constrained.

EXAMPLE 7

This example illustrates methods for MCT data analysis using additive frailty models.

Survival analysis are the most important endpoints of human clinical trials and can also be potentially adapted to MCT by first defining "clinical endpoint" of PFS (progress-free survival) and OS (overall survival) in preclinical setting. By the RECIST standard (Therasse P et al., J Natl Cancer Inst (2000) 92(3):205-16), we define PFS as the time until the starting tumor volume increases by 73%, and define OS as the time until the starting tumor volume triples, at which the tumor volume is usually between 300-600mm³, depending on initial tumor volumes. As we have shown in examples above, tumors already start exponential growth before it reaches 600mm³. The definitions are more of a custom and can certainly be adjusted otherwise. For example, Gao et al defined PFS as the time until tumor volume doubles (Gao H et al., Nat Med (2015) 21(11):1318-25). We can also define OS as the time until the starting tumor volume quadruples and so on.

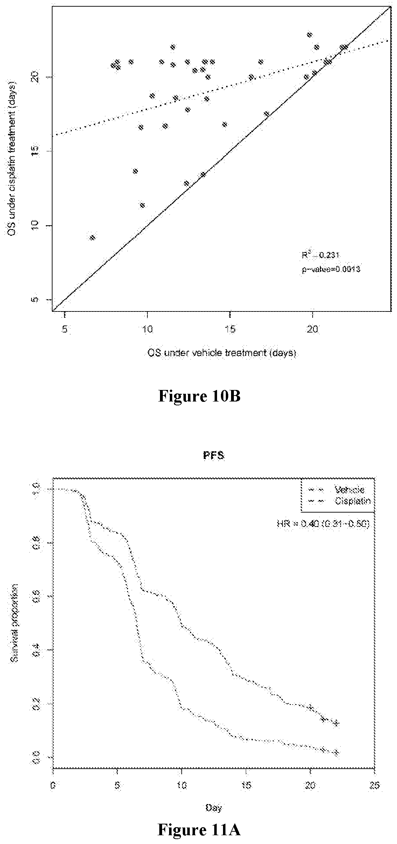

In human oncology trails, survival time of patients is usually assumed to be independent of each other. In MCT, this assumption does not hold. Mice have correlated survival time both within a PDX and between treatments (Figure 10A-B). There is heterogeneity on hazard and drug response for PDXs. We used an additive frailty model to fit the survival data in MCTs (Rondeau V et al., J Statistical Software (2012) 47(4): 1-28; Hanagal DD, Statistical Methods in Medical Research (2015) 24(6):936-36). In this model, the hazard function for the *j*-th mouse of the i-th PDX is

$$h_{ij}(t) = h_0(t)\exp(u_i + (w + v_i)T_{ij} + \beta^{\mathrm{T}}X_i) \qquad (10)$$

where $h_0(t)$ is the baseline hazard function. Parameter $u_i$ is the random effect (the first frailty term) associated with the i-th PDX that captures its characteristic growth, thus survival behavior, without drug treatment. Parameter $v_i$ is the random effect (the second frailty term) associated the i-th PDX that depicts its drug response deviating from the mean drug effect $w$ of all PDXs. $T_{ij}$ is the treatment variable and it equals 0 for the vehicle treatment and 1 for the drug treatment; $X_i$ is a vector for the PDX's covariates, e.g., cancer type and gene expression; $\beta^T$ is the parameter vector quantifying the fixed effects of the covariates. The two random effects $u_i$ and $v_i$ assume a bivariate normal distribution with zero means, variance $\sigma^2$ and $\tau^2$, and covariance $\rho\sigma\tau$. If the two random effects $u_i$ and $v_i$ are removed, the model reduces to the proportional hazard Cox model.

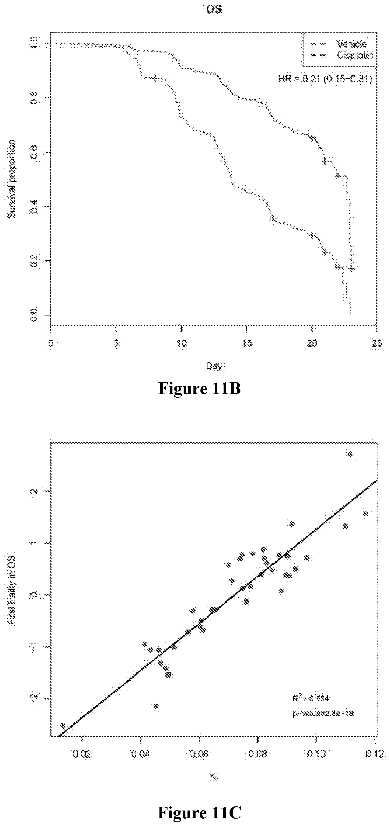

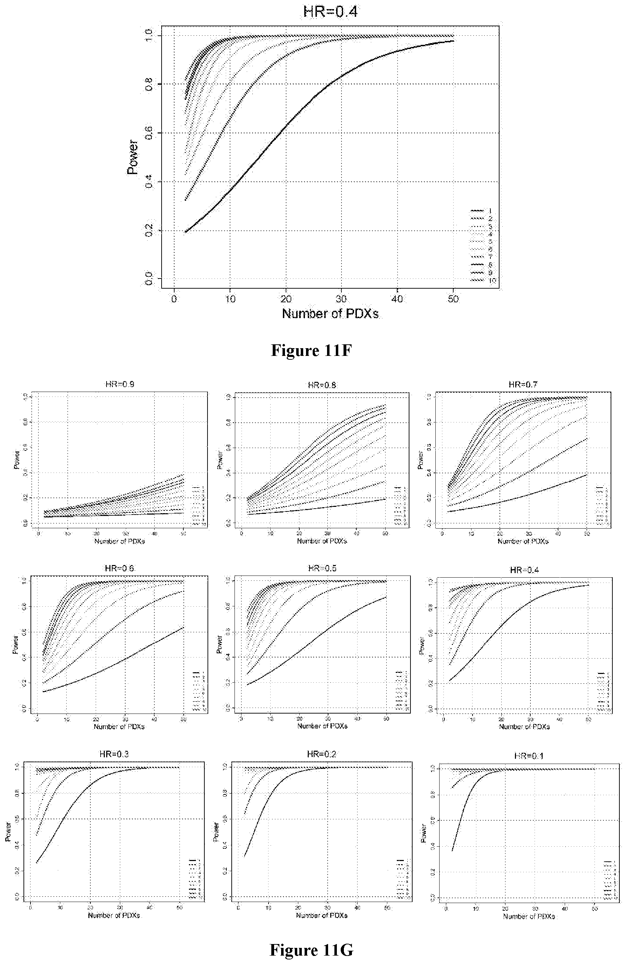

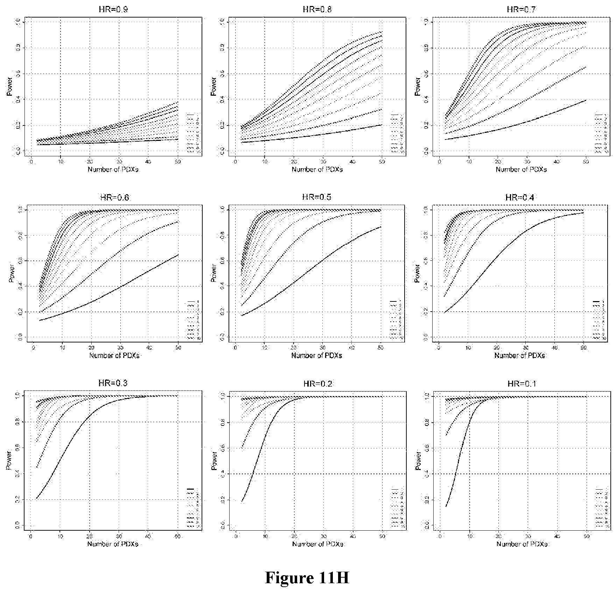

We used the cisplatin dataset in the previous example to illustrate modeling by the additive frailty model, using the R package frailtypack (Rondeau V et al., J Statistical Software (2012) 47(4):1-28). For simplicity, we used treatment as the sole covariate. By Equation 10, we observed that both frailty terms are significant larger than 0 (Wald test p-value<0.05) for PFS and OS analyses. Therefore, there is strong heterogeneity among PDXs and their drug response. The hazard ratio (HR) is estimated to be 0.40 (95% CI: 0.31-0.50) for PFS and 0.21 (95% CI: 0.15-0.31) for OS, both are sizably smaller than estimations under the proportional hazard Cox model, which gives PFS HR=0.48 (95% CI: 0.40-0.58) and OS HR=0.36 (95% CI: 0.28-0.46) (Figure 11A-B). These results show that without considering PDX heterogeneity, the drug effect can be severely underestimated.

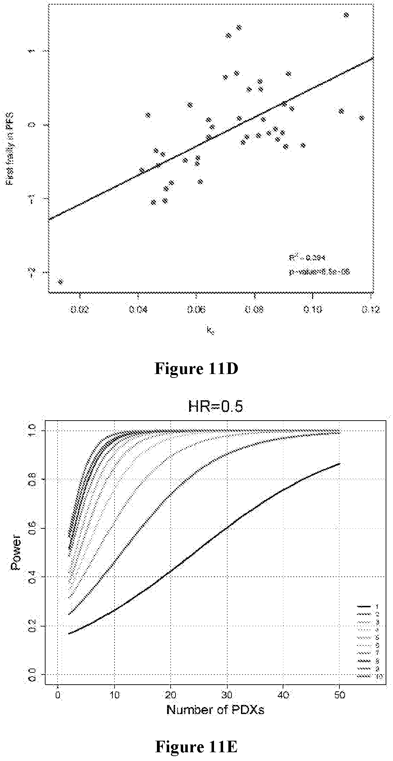

The first frailty term $u_i$ for a PDX measures its hazard of tumor progression. We observed that the estimated frailties from the OS survival analysis are negatively correlated with PDXs' growth rates in the vehicle group ($R^2 = 0.85$, **Figure 11C**). The fitting is much loose for the PFS survival analysis ($R^2 = 0.39$, **Figure 11D**), which can be explained by the somewhat irregular tumor growth compounded by relatively large measurement error at small tumor volumes.

We performed statistical power analysis for the survival analysis by assuming the n:n designs, and using parameters estimated from the cisplatin dataset with Weibull hazard functions (Figure 11E-H). We found that the survival analysis generally has lower power than LMM for comparable drug efficacy, and the 3:3 or above designs generally give much more power than the 1:1 and 2:2 designs.

The additive frailty model can readily be used for biomarker assessment and discovery by incorporating genomic status (e.g., mutation or expression) as a covariate. Because the frailty term already extracts PDX heterogeneity, the frailty model can be more accurate on quantifying the effect of a potential biomarker. Compared to LMM, survival analysis does not assume exponential or other forms of growth curves, and is therefore more flexible.

## References

1. Povlsen CO, Rygaard J. Heterotransplantation of human adenocarcinomas of the colon and rectum to the mouse mutant Nude. A study of nine consecutive transplantations. Acta pathologica et microbiologica Scandinavica Section A, Pathology 1971;79(2):159-69.

2. Castro JE. Human tumours grown in mice. Nature: New biology 1972;239(90):83-4.

3. Cobb LM. The behaviour of carcinoma of the large bowel in man following transplantation into immune deprived mice. British journal of cancer 1973;28(5):400-11.

4. Rosfjord E, Lucas J, Li G, Gerber HP. Advances in patient-derived tumor xenografts: from target identification to predicting clinical response rates in oncology. Biochemical pharmacology 2014;91(2):135-43.

5. Hidalgo M, Amant F, Biankin AV, Budinska E, Byrne AT, Caldas C, et al. Patient-derived xenograft models: an emerging platform for translational cancer research. Cancer discovery 2014;4(9):998-1013.

6. Gao H, Korn JM, Ferretti S, Monahan JE, Wang Y, Singh M, et al. High-throughput screening using patient-derived tumor xenografts to predict clinical trial drug response. Nature medicine 2015;21(11):1318-25.

7. Owonikoko TK, Zhang G, Kim HS, Stinson RM, Bechara R, Zhang C, et al. Patient-derived xenografts faithfully replicated clinical outcome in a phase II co-clinical trial of arsenic trioxide in relapsed small cell lung cancer. Journal of translational medicine 2016;14(1):111.

8. Guo S, Qian W, Cai J, Zhang L, Wery JP, Li Q. Molecular pathology of patient tumors, patient derived xenografts and cancer cell lines. Cancer research 2016.

9. Tentler JJ, Tan AC, Weekes CD, Jimeno A, Leong S, Pitts TM, et al. Patient-derived tumour xenografts as models for oncology drug development. Nat Rev Clin Oncol 2012;9(6):338-50.

10. DeRose YS, Wang G, Lin YC, Bernard PS, Buys SS, Ebbert MT, et al. Tumor grafts derived from women with breast cancer authentically reflect tumor pathology, growth, metastasis and disease outcomes. Nature medicine 2011;17(11):1514-20.

11. Zhang X, Claerhout S, Prat A, Dobrolecki LE, Petrovic I, Lai Q, et al. A renewable tissue resource of phenotypically stable, biologically and ethnically diverse, patient-derived human breast cancer xenograft models. Cancer research 2013;73(15):4885-97.

12. Zhao X, Liu Z, Yu L, Zhang Y, Baxter P, Voicu H, et al. Global gene expression profiling confirms the molecular fidelity of primary tumor-based orthotopic xenograft mouse models of medulloblastoma. Neuro-oncology 2012;14(5):574-83.

13. Kabos P, Finlay-Schultz J, Li C, Kline E, Finlayson C, Wisell J, et al. Patient-derived luminal breast cancer xenografts retain hormone receptor heterogeneity and help define unique estrogen-dependent gene signatures. Breast cancer research and treatment 2012;135(2):415-32.

14. Julien S, Merino-Trigo A, Lacroix L, Pocard M, Goere D, Mariani P, et al. Characterization of a large panel of patient-derived tumor xenografts representing the clinical heterogeneity of human colorectal cancer. Clinical cancer research : an official journal of the American Association for Cancer Research 2012;18(19):5314-28.

15. Malaney P, Nicosia SV, Dave V. One mouse, one patient paradigm: New avatars of personalized cancer therapy. Cancer letters 2014;344(1):1-12.

16. Smith PG, Sutton D, Bertotti A, Trusolino L, Airhart S, Ming ST, et al. Abstract 1191: Translational Proof-of-Concept (TransPoC), a not-for-profit research organization enabling access to large-scale translational oncology platforms: The Patient-Derived Xenograft network. Cancer research 2014;74(19 Supplement):1191-91.

17. Budinská E, Popovici V. The EurOPDX consortium: Sharing patient tumor-derived xenografts for collaborative multicentric preclinical trials. Molecular cancer therapeutics 2014;12(11_Supplement):A8-A8.

18. Chen D, Huang X, Cai J, Guo S, Qian W, Wery JP, et al. A set of defined oncogenic mutation alleles seems to better predict the response to cetuximab in CRC patient-derived xenograft than KRAS 12/13 mutations. Oncotarget 2015;6(38):40815-21.

19. Guo S, Chen D, Huang X, Cai J, Wery JP, Li QX. Cetuximab response in CRC patient-derived xenografts seems predicted by an expression based RAS pathway signature. Oncotarget 2016.

20. Guo S, Qian W, Cai J, Zhang L, Wery JP, Li QX. Molecular Pathology of Patient Tumors, Patient-Derived Xenografts, and Cancer Cell Lines. Cancer research 2016.

21. Zhang L, Yang J, Cai J, Song X, Deng J, Huang X, et al. A subset of gastric cancers with EGFR amplification and overexpression respond to cetuximab therapy. Sci Rep 2013;3:2992.

22. Teicher BA. Tumor models for preclinical development of targeted agents. Progress in drug research Fortschritte der Arzneimittelforschung Progres des recherches pharmaceutiques 2005;63:43-66.

23. Therasse P, Arbuck SG, Eisenhauer EA, Wanders J, Kaplan RS, Rubinstein L, et al. New guidelines to evaluate the response to treatment in solid tumors. European Organization for Research and Treatment of Cancer, National Cancer Institute of the United States, National Cancer Institute of Canada. J Natl Cancer Inst 2000;92(3):205-16.

24. Townsend EC, Murakami MA, Christodoulou A, Christie AL, Koster J, DeSouza TA, et al. The Public Repository of Xenografts Enables Discovery and Randomized Phase II-like Trials in Mice. Cancer cell 2016;29(4):574-86.

25. Heitjan DF, Manni A, Santen RJ. Statistical analysis of in vivo tumor growth experiments. Cancer research 1993;53(24):6042-50.

26. Wu J. Statistical inference for tumor growth inhibition T/C ratio. Journal of biopharmaceutical statistics 2010;20(5):954-64.

27. Laajala TD, Corander J, Saarinen NM, Makela K, Savolainen S, Suominen MI, et al. Improved statistical modeling of tumor growth and treatment effect in preclinical animal studies with highly heterogeneous responses in vivo. Clinical cancer research : an official journal of the American Association for Cancer Research 2012; 18(16):4385-96.

28. Zhao L, Morgan MA, Parsels LA, Maybaum J, Lawrence TS, Normolle D. Bayesian hierarchical changepoint methods in modeling the tumor growth profiles in xenograft experiments. Clinical cancer research : an official journal of the American Association for Cancer Research 2011;17(5): 1057-64.

29. Xia C, Wu J, Liang H. Model Tumor Pattern and Compare Treatment Effects Using Semiparametric Linear Mixed-Effects Models. Journal of Biometrics & Biostatistics 2013.

30. Pierrillas PB, Tod M, Amiel M, Chenel M, Henin E. Improvement of Parameter Estimations in Tumor Growth Inhibition Models on Xenografted Animals: Handling Sacrifice Censoring and Error Caused by Experimental Measurement on Larger Tumor Sizes. The AAPS journal 2016.

31. Choudhury KR, Kasman I, Plowman GD. Analysis of multi-arm tumor growth trials in xenograft animals using phase change adaptive piecewise quadratic models. Statistics in medicine 2010;29(23):2399-409.

32. Yang M, Shan B, Li Q, Song X, Cai J, Deng J, et al. Overcoming erlotinib resistance with tailored treatment regimen in patient-derived xenografts from naive Asian NSCLC patients. International journal of cancer Journal international du cancer 2013;132(2):E74-84.

33. Yang M, Xu X, Cai J, Ning J, Wery JP, Li QX. NSCLC harboring EGFR exon-20 insertions after the regulatory C-helix of kinase domain responds poorly to known EGFR inhibitors. International journal of cancer Journal international du cancer 2016;139(1):171-6.

34. Hinkley DV. On the Ratio of Two Correlated Normal Random Variables. Biometrika 1969;56(3):635-39.

35. Brody JP, Williams BA, Wold BJ, Quake SR. Significance and statistical errors in the analysis of DNA microarray data. Proceedings of the National Academy of Sciences of the United States of America 2002;99(20):12975-8.

36. Zhang L, Yang J, Cai J, Song X, Deng J, Huang X, et al. A subset of gastric cancers with EGFR amplification and overexpression respond to cetuximab therapy. Scientific reports 2013;3:2992.

37. Kumar P, Henikoff S, Ng PC. Predicting the effects of coding non-synonymous variants on protein function using the SIFT algorithm. Nature protocols 2009;4(7):1073-81.

38. Zhang X, Xu J, Liu H, Yang L, Liang J, Xu N, et al. Predictive biomarkers for the efficacy of cetuximab combined with cisplatin and capecitabine in advanced gastric or esophagogastric junction adenocarcinoma: a prospective multicenter phase 2 trial. Medical oncology 2014;31(10):226.

39. West BT, Welch KB, Galecki AT. Linear Mixed Models: A Practical Guide Using Statistical Software, Second Edition. Crc Press 2014.

40. De Dosso S, Zanellato E, Nucifora M, Boldorini R, Sonzogni A, Biffi R, et al. ERCC1 predicts outcome in patients with gastric cancer treated with adjuvant cisplatin-based chemotherapy. Cancer chemotherapy and pharmacology 2013;72(1):159-65.

41. Wang J, Zhou XQ, Li JY, Cheng JF, Zeng XN, Li X, et al. Prognostic significance of ERCC1 expression in postoperative patients with gastric cancer. Chinese journal of cancer research = Chung-kuo yen cheng yen chiu 2014;26(3):323-30.

42. Metzger R, Leichman CG, Danenberg KD, Danenberg PV, Lenz HJ, Hayashi K, et al. ERCC1 mRNA levels complement thymidylate synthase mRNA levels in predicting response and survival for gastric cancer patients receiving combination cisplatin and fluorouracil chemotherapy. Journal of clinical oncology : official journal of the American Society of Clinical Oncology 1998;16(1):309-16.

43. Kwon HC, Roh MS, Oh SY, Kim SH, Kim MC, Kim JS, et al. Prognostic value of expression of ERCC1, thymidylate synthase, and glutathione S-transferase P1 for 5-fluorouracil/oxaliplatin chemotherapy in advanced gastric cancer. Annals of oncology : official journal of the European Society for Medical Oncology / ESMO 2007;18(3):504-9.

44. Hirakawa M, Sato Y, Ohnuma H, Takayama T, Sagawa T, Nobuoka T, et al. A phase II study of neoadjuvant combination chemotherapy with docetaxel, cisplatin, and S-1 for locally advanced resectable gastric cancer: nucleotide excision repair (NER) as potential chemoresistance marker. Cancer chemotherapy and pharmacology 2013;71(3):789-97.

45. Miura JT, Xiu J, Thomas J, George B, Carron BR, Tsai S, et al. Tumor profiling of gastric and esophageal carcinoma reveal different treatment options. Cancer biology & therapy 2015;16(5):764-9.

46. Bamias A, Karina M, Papakostas P, Kostopoulos I, Bobos M, Vourli G, et al. A randomized phase III study of adjuvant platinum/docetaxel chemotherapy with or without radiation therapy in patients with gastric cancer. Cancer Chemotherapy & Pharmacology 2010;65(6):1009-21.

47. Baek SK, Kim SY, Lee JJ, Kim YW, Yoon HJ, Cho KS. Increased ERCC expression correlates with improved outcome of patients treated with cisplatin as an adjuvant therapy for curatively resected gastric cancer. Cancer Research & Treatment 2006;38(1):19-24.

48. Kim KH, Kwon HC, Oh SY, Kim SH, Lee S, Kwon KA, et al. Clinicopathologic significance of ERCC1, thymidylate synthase and glutathione S-transferase P1 expression for advanced gastric cancer patients receiving adjuvant 5-FU and cisplatin chemotherapy. Biomarkers : biochemical indicators of exposure, response, and susceptibility to chemicals 2011;16(1):74-82.

49. Sonnenblick A, Rottenberg Y, Kadouri L, Wygoda M, Rivkind A, Vainer GW, et al. Long-term outcome of continuous 5-fluorouracil/cisplatin-based chemotherapy followed by chemoradiation in patients with resected gastric cancer. Medical oncology 2012;29(5):3035-8.

50. Squires MH, 3rd, Fisher SB, Fisher KE, Patel SH, Kooby DA, El-Rayes BF, et al. Differential expression and prognostic value of ERCC1 and thymidylate synthase in resected gastric adenocarcinoma. Cancer 2013;119(17):3242-50.

51. Rondeau V, Mazroui Y, Gonzalez JR. frailtypack : An R Package for the Analysis of Correlated Survival Data with Frailty Models Using Penalized Likelihood Estimation or Parametrical Estimation. Journal of Statistical Software 2012;47(4):1-28.

52. Hanagal DD. Modeling survival data using frailty models. Statistical Methods in Medical Research 2015;24(6):936-36.

53. Benzekry S, Lamont C, Beheshti A, Tracz A, Ebos JM, Hlatky L, et al. Classical mathematical models for description and prediction of experimental tumor growth. PLoS computational biology 2014;10(8):e1003800.

54. Ferrer L, Rondeau V, Dignam J, Pickles T, Jacqmin-Gadda H, Proust-Lima C. Joint modelling of longitudinal and multi-state processes: application to clinical progressions in prostate cancer. Statistics in medicine 2016.

## Claims

1. A method of conducting mouse clinical trial comprising the steps of:

receiving a dataset of tumor volumes measured in a mouse clinical trial, wherein the mouse clinical trial comprises the steps of:

measuring tumor volume of a tumor sample of a treatment group and tumor volume of a control group at a plurality of days,

wherein a tumor sample has been obtained from a patient, and grafted to the treatment group comprising m mice and the control group comprising n mice, wherein m and n are integers,

wherein a drug has been administered to the treatment group and a vehicle has been administered to the control group; and

determining tumor growth curve of the treatment group and tumor growth curve of the control group;

determining area under curve (AUC) of the treatment group ($AUC_T$) and AUC of the control group ($AUC_C$); and

evaluating efficacy of the drug based on an AUC ratio between the $AUC_T$ and the $AUC_C$,

wherein the $$AUC_T = \int_0^{d(T)} \ln V_x^{(T)} \, dx - d(T) \times \ln V_0^{(T)},$$

wherein $V_0^{(T)}$ is the tumor volume of the treatment group at day 0, $V_x^{(T)}$ is the tumor volume of the treatment group at day x, d(T) is the number of days the measuring step for the treatment group lasts; and

wherein the $$AUC_C = \int_0^{d} \ln V_x^{(C)} \, dx - d \times \ln V_0^{(C)},$$

wherein $V_0^{(C)}$ is the tumor volume of the control group at day 0, $V_x^{(C)}$ is the tumor volume of the control group at day x, d(C) is the number of days the measuring step for the control group lasts,

wherein d(T) ≠ d(C), wherein the $$AUC \text{ ratio} = \frac{AUC_T/d(T)^2}{AUC_C/d(C)^2}.$$

2. The method of claim 1,

wherein $m \geq 3$ and $n \geq 3$; or
wherein m = n; or
wherein the evaluating step comprises determining that the drug is effective when the AUC ratio is less than 1; or
wherein the tumor volumes are between 100-300 mm$^3$.

3. The method of claim 1, further comprising the step of determining correlation of a factor to the efficacy of the drug.

4. The method of claim 3, wherein the factor is expression level of a gene.

5. The method of claim 4, wherein the gene is EGFR and the drug is cetuximab.

6. The method of claim 1, wherein the tumor sample is pre-treated before grafting to the treatment group or the control group.

7. The method of claim 1, wherein the tumor sample comprises a tumor cell or cancerous cell, and is grafted to the treatment group or the control group through subcutaneously injection.

8. The method of claim 1, wherein the drug used is a potential drug for treating tumor or cancer.

9. The method of claim 1, wherein the drug is administered intravenously, subcutaneously, orally, intramuscularly, intraventricularly, intragastrically, or by inhalation.

10. The method of claim 1, wherein the vehicle is a sham preparation (e.g., a placebo), either an excipient-only preparation or a sugar pill preparation.

11. The method of claim 1, wherein the tumor volume is measured every one, two, three or four days.

**Patentansprüche**

1. Verfahren zur Durchführung einer klinischen Mausstudie, welche die Schritte umfasst:

Empfangen eines Datensatzes von Tumorvolumina, die in einer klinischen Mausstudie gemessen wurden, wobei die klinische Mausstudie die Schritte umfasst:

Messen des Tumorvolumens einer Tumorprobe einer Behandlungsgruppe und des Tumorvolumens einer Kontrollgruppe an mehreren Tagen,

wobei die Tumorprobe von einem Patienten erhalten, und auf die Behandlungsgruppe, die m Mäuse umfasst, und die Kontrollgruppe transplantiert wurde, die n Mäuse umfasst,
wobei m und n ganze Zahlen sind,
wobei ein Medikament an die Behandlungsgruppe und ein Vehikel an die Kontrollgruppe verabreicht wurde;

Bestimmen der Tumorwachstumskurve der Behandlungsgruppe und der Tumorwachstumskurve der Kontrollgruppe;
Bestimmen der Fläche unter der Kurve (AUC) der Behandlungsgruppe ($AUC_T$) und der AUC der Kontrollgruppe ($AUC_C$); und
Bewertung der Wirksamkeit des Arzneimittels auf der Grundlage eines AUC-Verhältnisses zwischen der $AUC_T$ und der $AUC_C$,

wobei $AUC_T = \int_0^{d(T)} \ln V_x^{(T)}\, dx - d(T) \times \ln V_0^{(T)}$ ,

wobei $V_0^{(T)}$ das Tumorvolumen der Behandlungsgruppe am Tag 0 ist, $V_x^{(T)}$ das Tumorvolumen der Behandlungsgruppe am Tag x ist, d(T) die Anzahl der Tage ist, die der Messschritt für die Behandlungsgruppe dauert; und

wobei $AUC_C = \int_0^{d} \ln V_x^{(C)}\, dx - d \times \ln V_0^{(C)}$ ,

wobei $V_0^{(C)}$ das Tumorvolumen der Kontrollgruppe am Tag 0 ist, $V_x^{(C)}$ das Tumorvolumen der Kontrollgruppe am Tag x ist, d(C) die Anzahl der Tage ist, die der Messschritt für die Kontrollgruppe dauert,

wobei $d(T) \neq d(C)$, wobei das $AUC\text{-Verhältnis} = \dfrac{AUC_T/d(T)^2}{AUC_C/d(C)^2}$ .

2. Verfahren nach Anspruch 1,

wobei $m \geq 3$ und $n \geq 3$; oder
wobei m = n; oder
wobei der Bewertungsschritt die Bestimmung umfasst, dass das Arzneimittel wirksam ist, wenn das AUC-Verhältnis kleiner als 1 ist; oder
wobei die Tumorvolumina zwischen 100-300 $mm^3$ liegen.

3. Verfahren nach Anspruch 1, welches ferner den Schritt der Bestimmung der Korrelation eines Faktors mit der Wirksamkeit des Arzneimittels umfasst.

4. Verfahren nach Anspruch 3, worin der Faktor das Expressionsniveau eines Gens ist.

5. Verfahren nach Anspruch 4, worin das Gen EGFR ist und das Medikament Cetuximab ist.

6. Verfahren nach Anspruch 1, wobei die Tumorprobe vor dem Transplantieren in die Behandlungsgruppe oder die Kontrollgruppe vorbehandelt wird.

7. Verfahren nach Anspruch 1, wobei die Tumorprobe eine Tumorzelle oder Krebszelle umfasst und der Behandlungsgruppe oder der Kontrollgruppe durch subkutane Injektion transplantiert wird.

8. Verfahren nach Anspruch 1, worin das verwendete Medikament ein potentielles Medikament zur Behandlung eines Tumors oder Krebs ist.

9. Verfahren nach Anspruch 1, wobei das Medikament intravenös, subkutan, oral, intramuskulär, intraventrikulär, intragastrisch oder durch Inhalation verabreicht wird.

**10.** Verfahren nach Anspruch 1, worin das Vehikel ein Scheinpräparat (z.B. ein Placebo) ist, entweder ein reines Hilfsstoffpräparat oder ein Zuckerpillenpräparat.

**11.** Verfahren nach Anspruch 1, wobei das Tumorvolumen alle ein, zwei, drei oder vier Tage gemessen wird.

## Revendications

**1.** Méthode de conduite d'un essai clinique chez la souris comprenant les étapes suivantes:
recevoir un ensemble de données de volumes tumoraux mesurés dans un essai clinique sur des souris, l'essai clinique sur des souris comprenant les étapes suivantes:

mesurer le volume tumoral d'un échantillon de tumeur d'un groupe de traitement et le volume tumoral d'un groupe de contrôle à plusieurs jours,

dans lequel l'échantillon de tumeur a été obtenu à partir d'un patient, et greffé au groupe de traitement comprenant m souris et au groupe de contrôle comprenant n souris, où m et n sont des nombres entiers, dans lequel un médicament a été administré au groupe de traitement et un véhicule a été administré au groupe de contrôle;

déterminer la courbe de croissance tumorale du groupe de traitement et la courbe de croissance tumorale du groupe de contrôle;
déterminer l'aire sous la courbe (AUC) du groupe de traitement ($AUC_T$) et l'AUC du groupe de contrôle (AUCc); et
évaluer l'efficacité du médicament sur la base d'un rapport AUC entre l'$AUC_T$ et l'AUCc,

dans lequel $\mathrm{AUC_T} = \int_0^{d(T)} \ln V_x^{(T)} \, dx - \mathrm{d(T)} \times \ln V_0^{(T)}$ ,

dans lequel $V_0^{(T)}$ est le volume tumoral du groupe de traitement au jour 0, $V_x^{(T)}$ est le volume tumoral du groupe de traitement au jour x, d(T) est le nombre de jours que dure l'étape de mesure pour le groupe de traitement; et

dans lequel $\mathrm{AUC_C} = \int_0^{d} \ln V_x^{(C)} \, dx - \mathrm{d} \times \ln V_0^{(C)}$ ,

dans lequel $V_0^{(C)}$ est le volume tumoral du groupe de contrôle au jour 0, $V_x^{(C)}$ est le volume tumoral du groupe témoin au jour x, d(C) est le nombre de jours que dure l'étape de mesure pour le groupe de contrôle,

où d(T) ≠ d(C), où le rapport $\mathrm{AUC} = \dfrac{AUC_T/d(T)^2}{AUC_C/d(C)^2}$ .

**2.** Méthode de la revendication 1,

dans laquelle m ≥ 3 et n ≥ 3; ou
dans laquelle m = n; ou
dans laquelle l'étape d'évaluation consiste à déterminer que le médicament est efficace lorsque le rapport AUC est inférieur à 1; ou
dans laquelle les volumes tumoraux sont compris entre 100 et 300 mm$^3$.

**3.** Méthode de la revendication 1, comprenant en outre l'étape consistant à déterminer la corrélation d'un facteur avec l'efficacité du médicament.

**4.** Méthode de la revendication 3, dans laquelle le facteur est le niveau d'expression d'un gène.

**5.** Méthode de la revendication 4, dans laquelle le gène est l'EGFR et le médicament est le cetuximab.

**6.** Méthode de la revendication 1, dans laquelle l'échantillon de tumeur est prétraité avant d'être greffé au groupe de traitement ou au groupe de contrôle.

**7.** Méthode de la revendication 1, dans laquelle l'échantillon de tumeur comprend une cellule tumorale ou cancéreuse, et est greffé au groupe de traitement ou au groupe de contrôle par injection sous-cutanée.

8. Méthode de la revendication 1, dans laquelle le médicament utilisé est un médicament potentiel pour le traitement de la tumeur ou du cancer.

9. Méthode de la revendication 1, dans laquelle le médicament est administré par voie intraveineuse, sous-cutanée, orale, intramusculaire, intraventriculaire, intragastrique ou par inhalation.

10. Méthode de la revendication 1, dans laquelle le véhicule est une préparation fictive (par exemple, un placebo), soit une préparation à base d'excipient uniquement, soit une préparation sous forme de pilule de sucre.

11. Méthode de la revendication 1, dans laquelle le volume de la tumeur est mesuré tous les un, deux, trois ou quatre jours.

**Figure 1**

**Figure 2**

**Figure 3A**

**Figure 3B**

**Figure 4A**

**Figure 4B**

**Figure 4C**

**Figure 5A**

Figure 5B

Figure 5C

30

**Figure 5D**

**Figure 5E**

**Figure 5F**

**Figure 6A**

**Figure 6B**

**Figure 6C**

**Figure 7A**

**Figure 7B**

**Figure 7C**

**Figure 7D**

**Figure 7E**

**Figure 7F**

**Figure 7G**

**Figure 7H**

Figure 8A

Figure 8B

Figure 9A

Figure 9B

**Figure 9C**

**Figure 9D**

**Figure 9E**

Figure 9F

**Figure 9G**

**Figure 10A**

**Figure 10B**

**Figure 11A**

**Figure 11B**

**Figure 11C**

**Figure 11D**

HR=0.5

**Figure 11E**

Figure 11F

Figure 11G

**Figure 11H**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **POVLSEN CO ; RYGAARD J.** *Pathology,* 1971, vol. 79 (2), 159-69
- **CASTRO JE.** *Nature: New biology,* 1972, vol. 239 (90), 83-4
- **COBB LM.** *British journal of cancer,* 1973, vol. 28 (5), 400-11
- **ROSFJORD E et al.** *Biochemical pharmacology,* 2014, vol. 91 (2), 135-43
- **HIDALGO M et al.** *Cancer discovery,* 2014, vol. 4 (9), 998-1013
- **GAO H et al.** *Nat Med,* 2015, vol. 21 (11), 1318-25
- **OWONIKOKO TK et al.** *J Transl Med,* 2016, vol. 14 (1), 111
- **GUO S et al.** *Cancer Res,* 2016
- **TENTLER JJ et al.** *Nat Rev Clin Oncol,* 2012, vol. 9 (6), 338-50
- **DEROSE YS et al.** *Nat Med,* 2011, vol. 17 (11), 1514-20
- **ZHANG X et al.** *Cancer Res,* 2013, vol. 73 (15), 4885-97
- **ZHAO X et al.** *Neuro-oncology,* 2012, vol. 14 (5), 574-83
- **KABOS P et al.** *Breast Cancer Res and Treatment,* 2012, vol. 135 (2), 415-32
- **JULIEN S et al.** *Clinical Cancer Res,* 2012, vol. 18 (19), 5314-28
- **MALANEY P et al.** *Cancer Letters,* 2014, vol. 344 (1), 1-12
- **SMITH PG et al.** *Cancer Res,* 2014, vol. 74 (19), 1191-91
- **BUDINSKÁ E ; POPOVICI V.** *Mol Cancer Therapeutics,* 2014, vol. 12 (11), A8-A8
- **CHEN D et al.** *Oncotarget,* 2015, vol. 6 (38), 40815-21
- **GUO S et al.** *Oncotarget,* 2016
- **ZHANG L et al.** *Sci Rep,* 2013, vol. 3, 2992
- **TEICHER BA.** *Progress in Drug Res,* 2005, vol. 63, 43-66
- **HEITJAN DF et al.** *Cancer Res,* 1993, vol. 53 (24), 6042-50
- **WU J et al.** *J Biopharmaceutical Statistics,* 2010, vol. 20 (5), 954-64
- **LAAJALA TD et al.** *Clinical Cancer Res,* 2012, vol. 18 (16), 4385-96
- **ZHAO L et al.** *Clinical Cancer Res,* 2011, vol. 17 (5), 1057-64
- **XIA C et al.** *J Biometrics & Biostatistics,* 2013
- **PIERRILLAS PB et al.** *The AAPS J,* 2016
- **CHOUDHURY KR et al.** *Statistics in Med,* 2010, vol. 29 (23), 2399-409
- **VAN DER MAATERN ; HINTTON.** *J machine Learning Res,* 2008, 2579-2605
- **KRIJTHE J.** *CRAN,* 2015
- **ZHAO et al.** *Clinical cancer research,* 2011, vol. 17, 1057-1064
- **BENZEKRY et al.** *PloS computational biology,* 2014
- **HINKLEY DV.** *Biometrika,* 1969, vol. 56 (3), 635-39
- **BRODY JP et al.** *PNAS,* 2002, vol. 99 (20), 12975-8
- **ZHANG L et al.** *Scientific Reports,* 2013, vol. 3, 2992
- **ZHANG X et al.** *Medical Oncology,* 2014, vol. 31 (10), 226
- **WEST BT et al.** Linear Mixed Models: A Practical Guide Using Statistical Software. Crc Press, 2014
- **METZGER R et al.** *J Clinical Oncology,* 1998, vol. 16 (1), 309-16
- **KWON HC et al.** *Annals of Oncology,* 2007, vol. 18 (3), 504-9
- **HIRAKAWA M et al.** *Cancer Chemotherapy and Pharmacology,* 2013, vol. 71 (3), 789-97
- **MIURA JT et al.** *Cancer Biology & Therapy,* 2015, vol. 16 (5), 764-9
- **BAMIAS A et al.** *Cancer Chemotherapy & Pharmacology,* 2010, vol. 65 (6), 1009-21
- **BAEK SK et al.** *Cancer Research & Treatment,* 2006, vol. 38 (1), 19-24
- **KIM KH et al.** *Biomarkers,* 2011, vol. 16 (1), 74-82
- **SONNENBLICK A et al.** *Medical Oncology,* 2012, vol. 29 (5), 3035-8
- **SQUIRES MH et al.** *Cancer,* 2013, vol. 119 (17), 3242-50
- **THERASSE P et al.** *J Natl Cancer Inst,* 2000, vol. 92 (3), 205-16
- **RONDEAU V et al.** *J Statistical Software,* 2012, vol. 47 (4), 1-28
- **HANAGAL DD.** *Statistical Methods in Medical Research,* 2015, vol. 24 (6), 936-36

- **POVLSEN CO ; RYGAARD J.** Heterotransplantation of human adenocarcinomas of the colon and rectum to the mouse mutant Nude. A study of nine consecutive transplantations. *Acta pathologica et microbiologica Scandinavica Section A, Pathology,* 1971, vol. 79 (2), 159-69
- **CASTRO JE.** Human tumours grown in mice. *Nature: New biology,* 1972, vol. 239 (90), 83-4
- **COBB LM.** The behaviour of carcinoma of the large bowel in man following transplantation into immune deprived mice. *British journal of cancer,* 1973, vol. 28 (5), 400-11
- **ROSFJORD E ; LUCAS J ; LI G ; GERBER HP.** Advances in patient-derived tumor xenografts: from target identification to predicting clinical response rates in oncology. *Biochemical pharmacology,* 2014, vol. 91 (2), 135-43
- **HIDALGO M ; AMANT F ; BIANKIN AV ; BUDINSKA E ; BYRNE AT ; CALDAS C et al.** Patient-derived xenograft models: an emerging platform for translational cancer research. *Cancer discovery,* 2014, vol. 4 (9), 998-1013
- **GAO H ; KORN JM ; FERRETTI S ; MONAHAN JE ; WANG Y ; SINGH M et al.** High-throughput screening using patient-derived tumor xenografts to predict clinical trial drug response. *Nature medicine,* 2015, vol. 21 (11), 1318-25
- **OWONIKOKO TK ; ZHANG G ; KIM HS ; STINSON RM ; BECHARA R ; ZHANG C et al.** Patient-derived xenografts faithfully replicated clinical outcome in a phase II co-clinical trial of arsenic trioxide in relapsed small cell lung cancer. *Journal of translational medicine,* 2016, vol. 14 (1), 111
- **GUO S ; QIAN W ; CAI J ; ZHANG L ; WERY JP ; LI Q.** Molecular pathology of patient tumors, patient derived xenografts and cancer cell lines. *Cancer research,* 2016
- **TENTLER JJ ; TAN AC ; WEEKES CD ; JIMENO A ; LEONG S ; PITTS TM et al.** Patient-derived tumour xenografts as models for oncology drug development. *Nat Rev Clin Oncol,* 2012, vol. 9 (6), 338-50
- **DEROSE YS ; WANG G ; LIN YC ; BERNARD PS ; BUYS SS ; EBBERT MT et al.** Tumor grafts derived from women with breast cancer authentically reflect tumor pathology, growth, metastasis and disease outcomes. *Nature medicine,* 2011, vol. 17 (11), 1514-20
- **ZHANG X ; CLAERHOUT S ; PRAT A ; DOBROLECKI LE ; PETROVIC I ; LAI Q et al.** A renewable tissue resource of phenotypically stable, biologically and ethnically diverse, patient-derived human breast cancer xenograft models. *Cancer research,* 2013, vol. 73 (15), 4885-97
- **ZHAO X ; LIU Z ; YU L ; ZHANG Y ; BAXTER P ; VOICU H et al.** Global gene expression profiling confirms the molecular fidelity of primary tumor-based orthotopic xenograft mouse models of medulloblastoma. *Neuro-oncology,* 2012, vol. 14 (5), 574-83
- **KABOS P ; FINLAY-SCHULTZ J ; LI C ; KLINE E ; FINLAYSON C ; WISELL J et al.** Patient-derived luminal breast cancer xenografts retain hormone receptor heterogeneity and help define unique estrogen-dependent gene signatures. *Breast cancer research and treatment,* 2012, vol. 135 (2), 415-32
- **JULIEN S ; MERINO-TRIGO A ; LACROIX L ; POCARD M ; GOERE D ; MARIANI P et al.** Characterization of a large panel of patient-derived tumor xenografts representing the clinical heterogeneity of human colorectal cancer. *Clinical cancer research : an official journal of the American Association for Cancer Research,* 2012, vol. 18 (19), 5314-28
- **MALANEY P ; NICOSIA SV ; DAVE V.** One mouse, one patient paradigm: New avatars of personalized cancer therapy. *Cancer letters,* 2014, vol. 344 (1), 1-12
- **SMITH PG ; SUTTON D ; BERTOTTI A ; TRUSOLINO L ; AIRHART S ; MING ST et al.** Abstract 1191: Translational Proof-of-Concept (TransPoC), a not-for-profit research organization enabling access to large-scale translational oncology platforms: The Patient-Derived Xenograft network. *Cancer research,* 2014, vol. 74 (19), 1191-91
- **BUDINSKÁ E ; POPOVICI V.** The EurOPDX consortium: Sharing patient tumor-derived xenografts for collaborative multicentric preclinical trials. *Molecular cancer therapeutics,* 2014, vol. 12 (11), A8-A8
- **CHEN D ; HUANG X ; CAI J ; GUO S ; QIAN W ; WERY JP et al.** A set of defined oncogenic mutation alleles seems to better predict the response to cetuximab in CRC patient-derived xenograft than KRAS 12/13 mutations. *Oncotarget,* 2015, vol. 6 (38), 40815-21
- **GUO S ; CHEN D ; HUANG X ; CAI J ; WERY JP ; LI QX.** Cetuximab response in CRC patient-derived xenografts seems predicted by an expression based RAS pathway signature. *Oncotarget,* 2016
- **GUO S ; QIAN W ; CAI J ; ZHANG L ; WERY JP ; LI QX.** Molecular Pathology of Patient Tumors, Patient-Derived Xenografts, and Cancer Cell Lines. *Cancer research,* 2016
- **ZHANG L ; YANG J ; CAI J ; SONG X ; DENG J ; HUANG X et al.** A subset of gastric cancers with EGFR amplification and overexpression respond to cetuximab therapy. *Sci Rep,* 2013, vol. 3, 2992
- **TEICHER BA.** Tumor models for preclinical development of targeted agents. *Progress in drug research Fortschritte der Arzneimittelforschung Progres des recherches pharmaceutiques,* 2005, vol. 63, 43-66

- **THERASSE P ; ARBUCK SG ; EISENHAUER EA ; WANDERS J ; KAPLAN RS ; RUBINSTEIN L et al.** New guidelines to evaluate the response to treatment in solid tumors. European Organization for Research and Treatment of Cancer, National Cancer Institute of the United States, National Cancer Institute of Canada. *J Natl Cancer Inst,* 2000, vol. 92 (3), 205-16
- **TOWNSEND EC ; MURAKAMI MA ; CHRISTO-DOULOU A ; CHRISTIE AL ; KOSTER J ; DES-OUZA TA et al.** The Public Repository of Xenografts Enables Discovery and Randomized Phase II-like Trials in Mice. *Cancer cell,* 2016, vol. 29 (4), 574-86
- **HEITJAN DF ; MANNI A ; SANTEN RJ.** Statistical analysis of in vivo tumor growth experiments. *Cancer research,* 1993, vol. 53 (24), 6042-50
- **WU J.** Statistical inference for tumor growth inhibition T/C ratio. *Journal of biopharmaceutical statistics,* 2010, vol. 20 (5), 954-64
- **LAAJALA TD ; CORANDER J ; SAARINEN NM ; MAKELA K ; SAVOLAINEN S ; SUOMINEN MI et al.** Improved statistical modeling of tumor growth and treatment effect in preclinical animal studies with highly heterogeneous responses in vivo. *Clinical cancer research : an official journal of the American Association for Cancer Research,* 2012, vol. 18 (16), 4385-96
- **ZHAO L ; MORGAN MA ; PARSELS LA ; MAY-BAUM J ; LAWRENCE TS ; NORMOLLE D.** Bayesian hierarchical changepoint methods in modeling the tumor growth profiles in xenograft experiments. *Clinical cancer research : an official journal of the American Association for Cancer Research,* 2011, vol. 17 (5), 1057-64
- **XIA C ; WU J ; LIANG H.** Model Tumor Pattern and Compare Treatment Effects Using Semiparametric Linear Mixed-Effects Models. *Journal of Biometrics & Biostatistics,* 2013
- **PIERRILLAS PB ; TOD M ; AMIEL M ; CHENEL M ; HENIN E.** Improvement of Parameter Estimations in Tumor Growth Inhibition Models on Xenografted Animals: Handling Sacrifice Censoring and Error Caused by Experimental Measurement on Larger Tumor Sizes. *The AAPS journal,* 2016
- **CHOUDHURY KR ; KASMAN I ; PLOWMAN GD.** Analysis of multi-arm tumor growth trials in xenograft animals using phase change adaptive piecewise quadratic models. *Statistics in medicine,* 2010, vol. 29 (23), 2399-409
- **YANG M ; SHAN B ; LI Q ; SONG X ; CAI J ; DENG J et al.** Overcoming erlotinib resistance with tailored treatment regimen in patient-derived xenografts from naive Asian NSCLC patients. *International journal of cancer Journal international du cancer,* 2013, vol. 132 (2), E74-84
- **YANG M ; XU X ; CAI J ; NING J ; WERY JP ; LI QX.** NSCLC harboring EGFR exon-20 insertions after the regulatory C-helix of kinase domain responds poorly to known EGFR inhibitors. *International journal of cancer Journal international du cancer,* 2016, vol. 139 (1), 171-6
- **HINKLEY DV.** On the Ratio of Two Correlated Normal Random Variables. *Biometrika,* 1969, vol. 56 (3), 635-39
- **BRODY JP ; WILLIAMS BA ; WOLD BJ ; QUAKE SR.** Significance and statistical errors in the analysis of DNA microarray data. *Proceedings of the National Academy of Sciences of the United States of America,* 2002, vol. 99 (20), 12975-8
- **ZHANG L ; YANG J ; CAI J ; SONG X ; DENG J ; HUANG X et al.** A subset of gastric cancers with EGFR amplification and overexpression respond to cetuximab therapy. *Scientific reports,* 2013, vol. 3, 2992
- **KUMAR P ; HENIKOFF S ; NG PC.** Predicting the effects of coding non-synonymous variants on protein function using the SIFT algorithm. *Nature protocols,* 2009, vol. 4 (7), 1073-81
- **ZHANG X ; XU J ; LIU H ; YANG L ; LIANG J ; XU N et al.** Predictive biomarkers for the efficacy of cetuximab combined with cisplatin and capecitabine in advanced gastric or esophagogastric junction adenocarcinoma: a prospective multicenter phase 2 trial. *Medical oncology,* 2014, vol. 31 (10), 226
- **WEST BT ; WELCH KB ; GALECKI AT.** Linear Mixed Models: A Practical Guide Using Statistical Software. Crc Press, 2014
- **DE DOSSO S ; ZANELLATO E ; NUCIFORA M ; BOLDORINI R ; SONZOGNI A ; BIFFI R et al.** ERCC1 predicts outcome in patients with gastric cancer treated with adjuvant cisplatin-based chemotherapy. *Cancer chemotherapy and pharmacology,* 2013, vol. 72 (1), 159-65
- **WANG J ; ZHOU XQ ; LI JY ; CHENG JF ; ZENG XN ; LI X et al.** Prognostic significance of ERCC1 expression in postoperative patients with gastric cancer. *Chinese journal of cancer research = Chung-kuo yen cheng yen chiu,* 2014, vol. 26 (3), 323-30
- **METZGER R ; LEICHMAN CG ; DANENBERG KD ; DANENBERG PV ; LENZ HJ ; HAYASHI K et al.** ERCC1 mRNA levels complement thymidylate synthase mRNA levels in predicting response and survival for gastric cancer patients receiving combination cisplatin and fluorouracil chemotherapy. *Journal of clinical oncology : official journal of the American Society of Clinical Oncology,* 1998, vol. 16 (1), 309-16

- **KWON HC ; ROH MS ; OH SY ; KIM SH ; KIM MC ; KIM JS et al.** Prognostic value of expression of ERCC1, thymidylate synthase, and glutathione S-transferase P1 for 5-fluorouracil/oxaliplatin chemotherapy in advanced gastric cancer. *Annals of oncology : official journal of the European Society for Medical Oncology / ESMO,* 2007, vol. 18 (3), 504-9
- **HIRAKAWA M ; SATO Y ; OHNUMA H ; TAKAYAMA T ; SAGAWA T ; NOBUOKA T et al.** A phase II study of neoadjuvant combination chemotherapy with docetaxel, cisplatin, and S-1 for locally advanced resectable gastric cancer: nucleotide excision repair (NER) as potential chemoresistance marker. *Cancer chemotherapy and pharmacology,* 2013, vol. 71 (3), 789-97
- **MIURA JT ; XIU J ; THOMAS J ; GEORGE B ; CARRON BR ; TSAI S et al.** Tumor profiling of gastric and esophageal carcinoma reveal different treatment options. *Cancer biology & therapy,* 2015, vol. 16 (5), 764-9
- **BAMIAS A ; KARINA M ; PAPAKOSTAS P ; KOSTOPOULOS I ; BOBOS M ; VOURLI G et al.** A randomized phase III study of adjuvant platinum/docetaxel chemotherapy with or without radiation therapy in patients with gastric cancer. *Cancer Chemotherapy & Pharmacology,* 2010, vol. 65 (6), 1009-21
- **BAEK SK ; KIM SY ; LEE JJ ; KIM YW ; YOON HJ ; CHO KS.** Increased ERCC expression correlates with improved outcome of patients treated with cisplatin as an adjuvant therapy for curatively resected gastric cancer. *Cancer Research & Treatment,* 2006, vol. 38 (1), 19-24
- **KIM KH ; KWON HC ; OH SY ; KIM SH ; LEE S ; KWON KA et al.** Clinicopathologic significance of ERCC1, thymidylate synthase and glutathione S-transferase P1 expression for advanced gastric cancer patients receiving adjuvant 5-FU and cisplatin chemotherapy. *Biomarkers : biochemical indicators of exposure, response, and susceptibility to chemicals,* 2011, vol. 16 (1), 74-82
- **SONNENBLICK A ; ROTTENBERG Y ; KADOURI L ; WYGODA M ; RIVKIND A ; VAINER GW et al.** Long-term outcome of continuous 5-fluorouracil/cisplatin-based chemotherapy followed by chemoradiation in patients with resected gastric cancer. *Medical oncology,* 2012, vol. 29 (5), 3035-8
- **SQUIRES MH, 3RD ; FISHER SB ; FISHER KE ; PATEL SH ; KOOBY DA ; EL-RAYES BF et al.** Differential expression and prognostic value of ERCC1 and thymidylate synthase in resected gastric adenocarcinoma. *Cancer,* 2013, vol. 119 (17), 3242-50
- **RONDEAU V ; MAZROUI Y ; GONZALEZ JR.** frailtypack : An R Package for the Analysis of Correlated Survival Data with Frailty Models Using Penalized Likelihood Estimation or Parametrical Estimation. *Journal of Statistical Software,* 2012, vol. 47 (4), 1-28
- **HANAGAL DD.** Modeling survival data using frailty models. *Statistical Methods in Medical Research,* 2015, vol. 24 (6), 936-36
- **BENZEKRY S ; LAMONT C ; BEHESHTI A ; TRACZ A ; EBOS JM ; HLATKY L et al.** Classical mathematical models for description and prediction of experimental tumor growth. *PLoS computational biology,* 2014, vol. 10 (8), e1003800
- **FERRER L ; RONDEAU V ; DIGNAM J ; PICKLES T ; JACQMIN-GADDA H ; PROUST-LIMA C.** Joint modelling of longitudinal and multi-state processes: application to clinical progressions in prostate cancer. *Statistics in medicine,* 2016